(19) Europäisches Patentamt
European Patent Office
Office européen des brevets

(11) EP 4 512 401 A1

(12) **EUROPEAN PATENT APPLICATION**
published in accordance with Art. 153(4) EPC

(43) Date of publication:
**26.02.2025 Bulletin 2025/09**

(21) Application number: **23791907.1**

(22) Date of filing: **19.04.2023**

(51) International Patent Classification (IPC):
***A61K 31/44*** *(2006.01)* ***A61K 31/519*** *(2006.01)*
***A61K 45/00*** *(2006.01)* ***A61P 35/00*** *(2006.01)*
***A61P 35/02*** *(2006.01)* ***A61P 43/00*** *(2006.01)*

(52) Cooperative Patent Classification (CPC):
**A61K 31/44; A61K 31/519; A61K 45/00; A61P 35/00; A61P 35/02; A61P 43/00**

(86) International application number:
**PCT/JP2023/015683**

(87) International publication number:
**WO 2023/204259 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.04.2022 JP 2022069320**
**16.11.2022 EP 22207829**

(71) Applicants:
- **CHUGAI SEIYAKU KABUSHIKI KAISHA**
  **Tokyo 115-8543 (JP)**
- **F. Hoffmann-La Roche AG**
  **4070 Basel (CH)**

(72) Inventors:
- **HASEGAWA Masami**
  **Kamakura-shi, Kanagawa 247-8530 (JP)**
- **SASE Hitoshi**
  **Kamakura-shi, Kanagawa 247-8530 (JP)**
- **TANAKA, Hiroshi**
  **Kamakura-shi, Kanagawa 247-8530 (JP)**
- **SAKATA, Kiyoaki**
  **deceased (JP)**

(74) Representative: **Vossius & Partner Patentanwälte Rechtsanwälte mbB**
**Siebertstrasse 3**
**81675 München (DE)**

(54) **PHARMACEUTICAL FOR TREATING OR PREVENTING CANCER**

(57) The present invention provides a drug for treating or preventing cancer, the drug comprising a compound represented by the following formula (1), a salt thereof or a solvate thereof and being used in combination with a molecular-targeted agent, wherein the molecular-targeted agent is at least one member selected from the group consisting of a KRAS-G12C selective inhibitor, a SHP2 inhibitor, a VEGF inhibitor and a PD-1 axis binding antagonist.

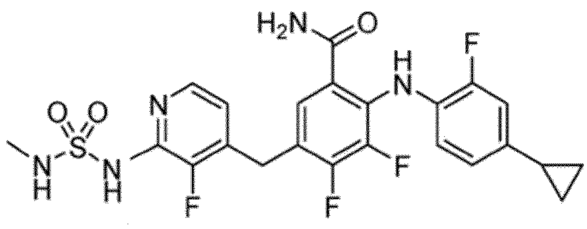

(1)

EP 4 512 401 A1

# Fig.19A

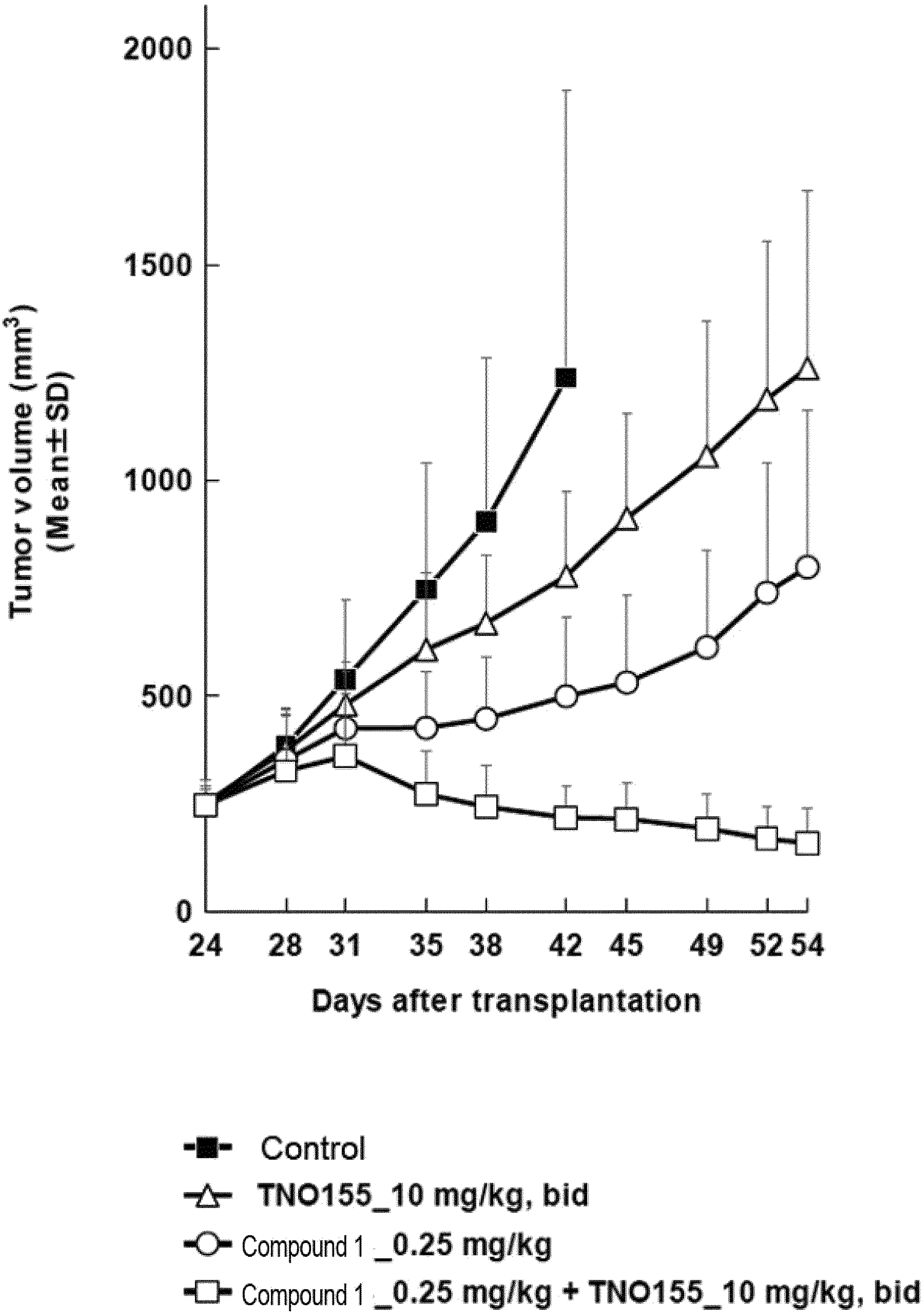
2000
1500
1000
500
0
Tumor volume (mm$^3$)
(Mean± SD)
24
28
31
35
38
42
45
49
52
54
Days after transplantation
Control
TNO155_10 mg/kg, bid
Compound 1 _0.25 mg/kg
Compound 1 _0.25 mg/kg + TNO155_10 mg/kg, bid

## Description

Technical Field

The present invention relates to a drug for treating or preventing cancer.

Background Art

Cancers are diseases that cause uncontrolled proliferation of cells due to gene abnormality. Methods for medically treating cancers comprise chemotherapy, and various anticancer agents are used. In recent years, many molecular-targeted agents which act specifically on molecules specifically expressed in certain cancer cells or molecules overexpressed in cancer cells have been developed as anticancer agents. For example, antibody drugs such as cetuximab, bevacizumab, and panitumumab (Patent Literature 1), small-molecule drugs such as gefitinib, erlotinib, and afatinib (Patent Literature 2), and arylamide derivatives having antitumor activity (Patent Literature 3) are known as molecular-targeted agents.

Citation List

Patent Literature

Patent Literature 1: Japanese Patent Laid-Open No. 2018-076369
Patent Literature 2: Japanese Patent Laid-Open No. 2021-063014
Patent Literature 3: International Publication No. WO 2021/149776

Non Patent Literature

Non Patent Literature 1: S.I. Ou, et al., "A12 The SHP2 Inhibitor RMC-4630 in Patients with KRAS-Mutant Non-Small Cell Lung Cancer: Preliminary Evaluation of a First-in-Man Phase 1 Clinical Trial", Journal of Thoracic Oncology Volume 15, Issue 2, Supplement, February 2020, P. S15-S16

Summary of Invention

Technical Problem

Under these circumstances, drugs excellent in drug efficacy are currently desired for the treatment or prevention of a cancer.

Accordingly, an object of the present invention is to provide a drug that exerts excellent effect on the treatment or prevention of a cancer.

Solution to Problem

The present invention encompasses the following [1] to [130].

[1] A drug for treating or preventing cancer, the drug comprising a compound represented by the following formula (1) (also referred to as a "compound 1"), a salt thereof or a solvate thereof and being used in combination with a molecular-targeted agent.

[Formula 1]

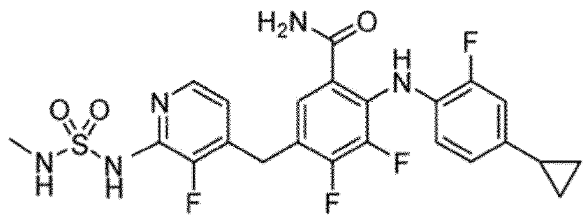

(1)

[2] A drug for treating or preventing cancer, the drug comprising a molecular-targeted agent and being used in combination with a compound 1, a salt thereof or a solvate thereof.

[3] The drug according to [1] or [2], wherein the compound 1, the salt thereof or the solvate thereof and the molecular-targeted agent are provided as a kit.

[4] A method for treating or preventing a cancer, comprising administering a compound 1, a salt thereof or a solvate thereof and a molecular-targeted agent to a subject.

[5] A compound 1, a salt thereof or a solvate thereof for use in the treatment or prevention of a cancer, the compound 1, the salt thereof or the solvate thereof being used in combination with a molecular-targeted agent.

[6] A molecular-targeted agent for use in the treatment or prevention of a cancer, the molecular-targeted agent being used in combination with a compound 1, a salt thereof or a solvate thereof.

[7] Use of a compound 1, a salt thereof or a solvate thereof for producing a drug for treating or preventing cancer, the drug being used in combination with a molecular-targeted agent.

[8] Use of a molecular-targeted agent for producing a drug for treating or preventing cancer, the drug being used in combination with a compound 1, a salt thereof or a solvate thereof.

[9] The drug, the method, the compound 1, the salt thereof or the solvate thereof for use, the molecular-targeted agent for use, or the use according to any of [1] to [8], wherein the compound 1, the salt thereof or the solvate thereof and the molecular-targeted agent are used in combination concurrently or separately.

[10] The drug, the method, the compound 1, the salt thereof or the solvate thereof for use, the molecular-targeted agent for use, or the use according to any of [1] to [9], wherein the compound 1, the salt thereof or the solvate thereof and the molecular-targeted agent are administered as a combination drug.

[11] The drug, the method, the compound 1, the salt thereof or the solvate thereof for use, the molecular-targeted agent for use, or the use according to any of [1] to [10], wherein the molecular-targeted agent is a MAPK/ERK pathway inhibitor.

[12] The drug, the method, the compound 1, the salt thereof or the solvate thereof for use, the molecular-targeted agent for use, or the use according to any of [1] to [11], wherein the molecular-targeted agent is at least one member selected from the group consisting of a KRAS inhibitor, a SHP2 inhibitor, a VEGF inhibitor and a PD-1 axis binding antagonist.

[13] The drug, the method, the compound 1, the salt thereof or the solvate thereof for use, the molecular-targeted agent for use, or the use according to any of [1] to [12], wherein the molecular-targeted agent is a KRAS inhibitor, a SHP2 inhibitor, a VEGF inhibitor or a PD-1 axis binding antagonist.

[14] The drug, the method, the compound 1, the salt thereof or the solvate thereof for use, the molecular-targeted agent for use, or the use according to [12] or [13], wherein the KRAS inhibitor is a KRAS-G12C selective inhibitor.

[15] The drug, the method, the compound 1, the salt thereof or the solvate thereof for use, the molecular-targeted agent for use, or the use according to [14], wherein the KRAS-G12C selective inhibitor is at least one member selected from the group consisting of sotorasib, adagrasib, a salt thereof, and a solvate thereof.

[16] The drug, the method, the compound 1, the salt thereof or the solvate thereof for use, the molecular-targeted agent for use, or the use according to [14] or [15], wherein the KRAS-G12C selective inhibitor is adagrasib, a salt thereof or a solvate thereof.

[17] The drug, the method, the compound 1, the salt thereof or the solvate thereof for use, the molecular-targeted agent for use, or the use according to [14] or [15], wherein the KRAS-G12C selective inhibitor is sotorasib, a salt thereof or a solvate thereof.

[18] The drug, the method, the compound 1, the salt thereof or the solvate thereof for use, the molecular-targeted agent for use, or the use according to [14], [15] or [17], wherein the KRAS-G12C selective inhibitor is sotorasib.

[19] The drug, the method, the compound 1, the salt thereof or the solvate thereof for use, the molecular-targeted agent for use, or the use according to any of [12] to [18], wherein the SHP2 inhibitor is at least one member selected from the group consisting of RMC-4550, TNO155, SHP099, NSC-87877, RMC-4630, GDC-1971, a salt thereof, and a solvate thereof.

[20] The drug, the method, the compound 1, the salt thereof or the solvate thereof for use, the molecular-targeted agent for use, or the use according to any of [12] to [19], wherein the SHP2 inhibitor is at least one member selected from the group consisting of RMC-4550, TNO155, GDC-1971, a salt thereof, and a solvate thereof.

[21] The drug, the method, the compound 1, the salt thereof or the solvate thereof for use, the molecular-targeted agent for use, or the use according to any of [12] to [20], wherein the SHP2 inhibitor is RMC-4550, a salt thereof or a solvate thereof.

[22] The drug, the method, the compound 1, the salt thereof or the solvate thereof for use, the molecular-targeted agent for use, or the use according to any of [12] to [20], wherein the SHP2 inhibitor is TNO155, a salt thereof or a solvate thereof.

[23] The drug, the method, the compound 1, the salt thereof or the solvate thereof for use, the molecular-targeted agent for use, or the use according to any of [12] to [20], wherein the SHP2 inhibitor is GDC-1971, a salt thereof or a

solvate thereof.

[24] The drug, the method, the compound 1, the salt thereof or the solvate thereof for use, the molecular-targeted agent for use, or the use according to any of [12] to [23], wherein the VEGF inhibitor is bevacizumab.

[25] The drug, the method, the compound 1, the salt thereof or the solvate thereof for use, the molecular-targeted agent for use, or the use according to any of [12] to [24], wherein the PD-1 axis binding antagonist is an anti-PD-L1 antibody.

[26] The drug, the method, the compound 1, the salt thereof or the solvate thereof for use, the molecular-targeted agent for use, or the use according to any of [1] to [13], wherein the molecular-targeted agent is a KRAS inhibitor.

[27] The drug, the method, the compound 1, the salt thereof or the solvate thereof for use, the molecular-targeted agent for use, or the use according to [26], wherein the KRAS inhibitor is a KRAS-G12C selective inhibitor.

[28] The drug, the method, the compound 1, the salt thereof or the solvate thereof for use, the molecular-targeted agent for use, or the use according to [27], wherein the KRAS-G12C selective inhibitor is at least one member selected from the group consisting of sotorasib, adagrasib, a salt thereof, and a solvate thereof.

[29] The drug, the method, the compound 1, the salt thereof or the solvate thereof for use, the molecular-targeted agent for use, or the use according to [27] or [28], wherein the KRAS-G12C selective inhibitor is adagrasib, a salt thereof or a solvate thereof.

[30] The drug, the method, the compound 1, the salt thereof or the solvate thereof for use, the molecular-targeted agent for use, or the use according to [27] or [28], wherein the KRAS-G12C selective inhibitor is sotorasib, a salt thereof or a solvate thereof.

[31] The drug, the method, the compound 1, the salt thereof or the solvate thereof for use, the molecular-targeted agent for use, or the use according to [27], [28] or [30], wherein the KRAS-G12C selective inhibitor is sotorasib.

[32] The drug, the method, the compound 1, the salt thereof or the solvate thereof for use, the molecular-targeted agent for use, or the use according to any of [1] to [13], wherein the molecular-targeted agent is a SHP2 inhibitor.

[33] The drug, the method, the compound 1, the salt thereof or the solvate thereof for use, the molecular-targeted agent for use, or the use according to [32], wherein the SHP2 inhibitor is at least one member selected from the group consisting of RMC-4550, TNO155, SHP099, NSC-87877, RMC-4630, GDC-1971, a salt thereof, and a solvate thereof.

[34] The drug, the method, the compound 1, the salt thereof or the solvate thereof for use, the molecular-targeted agent for use, or the use according to [32] or [33], wherein the SHP2 inhibitor is at least one member selected from the group consisting of RMC-4550, TNO155, GDC-1971, a salt thereof, and a solvate thereof.

[35] The drug, the method, the compound 1, the salt thereof or the solvate thereof for use, the molecular-targeted agent for use, or the use according to any of [32] to [34], wherein the SHP2 inhibitor is RMC-4550, a salt thereof or a solvate thereof.

[36] The drug, the method, the compound 1, the salt thereof or the solvate thereof for use, the molecular-targeted agent for use, or the use according to any of [32] to [34], wherein the SHP2 inhibitor is TNO155, a salt thereof or a solvate thereof.

[37] The drug, the method, the compound 1, the salt thereof or the solvate thereof for use, the molecular-targeted agent for use, or the use according to any of [32] to [34], wherein the SHP2 inhibitor is GDC-1971, a salt thereof or a solvate thereof.

[38] The drug, the method, the compound 1, the salt thereof or the solvate thereof for use, the molecular-targeted agent for use, or the use according to any of [1] to [13], wherein the molecular-targeted agent is a VEGF inhibitor.

[39] The drug, the method, the compound 1, the salt thereof or the solvate thereof for use, the molecular-targeted agent for use, or the use according to [38], wherein the VEGF inhibitor is bevacizumab.

[40] The drug, the method, the compound 1, the salt thereof or the solvate thereof for use, the molecular-targeted agent for use, or the use according to any of [1] to [13], wherein the molecular-targeted agent is a PD-1 axis binding antagonist.

[41] The drug, the method, the compound 1, the salt thereof or the solvate thereof for use, the molecular-targeted agent for use, or the use according to [40], wherein the PD-1 axis binding antagonist is an anti-PD-L1 antibody.

[42] The drug, the method, the compound 1, the salt thereof or the solvate thereof for use, the molecular-targeted agent for use, or the use according to any of [1] to [41], wherein the compound 1, the salt thereof or the solvate thereof is the salt of the compound 1.

[43] The drug, the method, the compound 1, the salt thereof or the solvate thereof for use, the molecular-targeted agent for use, or the use according to [42], wherein the salt is a sodium salt.

[44] The drug, the method, the compound 1, the salt thereof or the solvate thereof for use, the molecular-targeted agent for use, or the use according to any of [1] to [14], [19] to [27], wherein the compound 1, the salt thereof or the solvate thereof is a sodium salt of the compound 1, and the molecular-targeted agent is a KRAS-G12C selective inhibitor.

[45] The drug, the method, the compound 1, the salt thereof or the solvate thereof for use, the molecular-targeted

agent for use, or the use according to [44], wherein the KRAS-G12C selective inhibitor is sotorasib, a salt thereof or a solvate thereof.

[46] The drug, the method, the compound 1, the salt thereof or the solvate thereof for use, the molecular-targeted agent for use, or the use according to [44], wherein the KRAS-G12C selective inhibitor is adagrasib, a salt thereof or a solvate thereof.

[47] The drug, the method, the compound 1, the salt thereof or the solvate thereof for use, the molecular-targeted agent for use, or the use according to any of [1] to [21], [24], [25], [32] to [35], wherein the compound 1, the salt thereof or the solvate thereof is a sodium salt of the compound 1, and the molecular-targeted agent is RMC-4550, a salt thereof or a solvate thereof.

[48] The drug, the method, the compound 1, the salt thereof or the solvate thereof for use, the molecular-targeted agent for use, or the use according to any of [1] to [20], [22], [24], [25], [32] to [34], [36], wherein the compound 1, the salt thereof or the solvate thereof is a sodium salt of the compound 1, and the molecular-targeted agent is TNO155, a salt thereof or a solvate thereof.

[49] The drug, the method, the compound 1, the salt thereof or the solvate thereof for use, the molecular-targeted agent for use, or the use according to any of [1] to [20], [23] to [25], [32] to [34], [37], wherein the compound 1, the salt thereof or the solvate thereof is a sodium salt of the compound 1, and the molecular-targeted agent is GDC-1971, a salt thereof or a solvate thereof.

[50] The drug, the method, the compound 1, the salt thereof or the solvate thereof for use, the molecular-targeted agent for use, or the use according to any of [1] to [25], [38], [39], wherein the compound 1, the salt thereof or the solvate thereof is a sodium salt of the compound 1, and the molecular-targeted agent is bevacizumab.

[51] The drug, the method, the compound 1, the salt thereof or the solvate thereof for use, the molecular-targeted agent for use, or the use according to any of [1] to [25], [40], [41], wherein the compound 1, the salt thereof or the solvate thereof is a sodium salt of the compound 1, and the molecular-targeted agent is an anti-PD-L1 antibody.

[52] The drug, the method, the compound 1, the salt thereof or the solvate thereof for use, the molecular-targeted agent for use, or the use according to any of [1] to [41], wherein the compound 1, the salt thereof or the solvate thereof is the compound 1.

[53] The drug, the method, the compound 1, the salt thereof or the solvate thereof for use, the molecular-targeted agent for use, or the use according to any of [1] to [14], [19] to [27], wherein the compound 1, the salt thereof or the solvate thereof is the compound 1, and the molecular-targeted agent is the KRAS-G12C selective inhibitor.

[54] The drug, the method, the compound 1, the salt thereof or the solvate thereof for use, the molecular-targeted agent for use, or the use according to [53], wherein the KRAS-G12C selective inhibitor is sotorasib, a salt thereof or a solvate thereof.

[55] The drug, the method, the compound 1, the salt thereof or the solvate thereof for use, the molecular-targeted agent for use, or the use according to [53], wherein the KRAS-G12C selective inhibitor is adagrasib, a salt thereof or a solvate thereof.

[56] The drug, the method, the compound 1, the salt thereof or the solvate thereof for use, the molecular-targeted agent for use, or the use according to any of [1] to [21], [24], [25], [32] to [35], wherein the compound 1, the salt thereof or the solvate thereof is the compound 1, and the molecular-targeted agent is RMC-4550, a salt thereof or a solvate thereof.

[57] The drug, the method, the compound 1, the salt thereof or the solvate thereof for use, the molecular-targeted agent for use, or the use according to any of [1] to [20], [22], [24], [25], [32] to [34], [36], wherein the compound 1, the salt thereof or the solvate thereof is the compound 1, and the molecular-targeted agent is TNO155, a salt thereof or a solvate thereof.

[58] The drug, the method, the compound 1, the salt thereof or the solvate thereof for use, the molecular-targeted agent for use, or the use according to any of [1] to [20], [23] to [25], [32] to [34], [37], wherein the compound 1, the salt thereof or the solvate thereof is the compound 1, and the molecular-targeted agent is GDC-1971, a salt thereof or a solvate thereof.

[59] The drug, the method, the compound 1, the salt thereof or the solvate thereof for use, the molecular-targeted agent for use, or the use according to any of [1] to [25], [38], [39], wherein the compound 1, the salt thereof or the solvate thereof is the compound 1, and the molecular-targeted agent is bevacizumab.

[60] The drug, the method, the compound 1, the salt thereof or the solvate thereof for use, the molecular-targeted agent for use, or the use according to any of [1] to [25], [40], [41], wherein the compound 1, the salt thereof or the solvate thereof is the compound 1, and the molecular-targeted agent is an anti-PD-L1 antibody.

[61] The drug, the method, the compound 1, the salt thereof or the solvate thereof for use, the molecular-targeted agent for use, or the use according to any of [1] to [60], wherein the cancer is solid cancer or blood cancer.

[62] The drug, the method, the compound 1, the salt thereof or the solvate thereof for use, the molecular-targeted agent for use, or the use according to any of [1] to [61], wherein the cancer is at least one member selected from the group consisting of lung cancer, esophageal cancer, stomach cancer, colorectal cancer, uterine cancer, ovarian

cancer, pancreatic cancer, bladder cancer, thyroid gland cancer, skin cancer, head and neck cancer, leukemia, malignant lymphoma and multiple myeloma.

[63] The drug, the method, the compound 1, the salt thereof or the solvate thereof for use, the molecular-targeted agent for use, or the use according to any of [1] to [62], wherein the cancer is a cancer related to abnormality in RAS gene or MAPK/ERK pathway.

[64] The drug, the method, the compound 1, the salt thereof or the solvate thereof for use, the molecular-targeted agent for use, or the use according to [63], wherein the abnormality in MAPK/ERK pathway is abnormal activation of the MAPK/ERK pathway.

[65] The drug, the method, the compound 1, the salt thereof or the solvate thereof for use, the molecular-targeted agent for use, or the use according to [63] or [64], wherein the abnormality in MAPK/ERK pathway is abnormal activation due to amplification of a protein in the MAPK/ERK pathway.

[66] The drug, the method, the compound 1, the salt thereof or the solvate thereof for use, the molecular-targeted agent for use, or the use according to any of [63] to [65], wherein the abnormality in MAPK/ERK pathway is abnormal activation of the MAPK/ERK pathway due to gene mutation.

[67] The drug, the method, the compound 1, the salt thereof or the solvate thereof for use, the molecular-targeted agent for use, or the use according to [63], wherein the abnormality in MAPK/ERK pathway is abnormality in RAF gene.

[68] A drug for treating or preventing cancer, the drug comprising a compound represented by the following formula (1) (also referred to as a "compound 1"), a salt thereof or a solvate thereof and being used in combination with at least one member selected from the group consisting of a KRAS-G12C selective inhibitor, a SHP2 inhibitor, a VEGF inhibitor and a PD-1 axis binding antagonist.

[Formula 2]

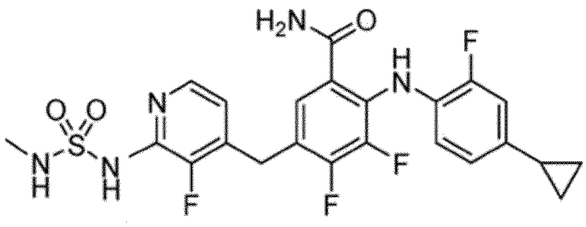

(1)

[69] A drug for treating or preventing cancer, the drug comprising at least one member selected from the group consisting of a KRAS-G12C selective inhibitor, a SHP2 inhibitor, a VEGF inhibitor and a PD-1 axis binding antagonist and being used in combination with a compound 1, a salt thereof or a solvate thereof.

[70] The drug according to [68] or [69], wherein the compound 1, the salt thereof or the solvate thereof and the at least one member selected from the group consisting of a KRAS-G12C selective inhibitor, a SHP2 inhibitor, a VEGF inhibitor and a PD-1 axis binding antagonist are provided as a kit.

[71] A method for treating or preventing a cancer, comprising administering a compound 1, a salt thereof or a solvate thereof and at least one member selected from the group consisting of a KRAS-G12C selective inhibitor, a SHP2 inhibitor, a VEGF inhibitor and a PD-1 axis binding antagonist to a subject.

[72] A compound 1, a salt thereof or a solvate thereof for use in the treatment or prevention of a cancer, the compound 1, the salt thereof or the solvate thereof being used in combination with at least one member selected from the group consisting of a KRAS-G12C selective inhibitor, a SHP2 inhibitor, a VEGF inhibitor and a PD-1 axis binding antagonist.

[73] At least one member selected from the group consisting of a KRAS-G12C selective inhibitor, a SHP2 inhibitor, a VEGF inhibitor and a PD-1 axis binding antagonist for use in the treatment or prevention of a cancer, the at least one member selected from the group consisting of a KRAS-G12C selective inhibitor, a SHP2 inhibitor, a VEGF inhibitor and a PD-1 axis binding antagonist being used in combination with a compound 1, a salt thereof or a solvate thereof.

[74] Use of a compound 1, a salt thereof or a solvate thereof for producing a drug for treating or preventing cancer, the drug being used in combination with at least one member selected from the group consisting of a KRAS-G12C selective inhibitor, a SHP2 inhibitor, a VEGF inhibitor and a PD-1 axis binding antagonist.

[75] Use of at least one member selected from the group consisting of a KRAS-G12C selective inhibitor, a SHP2 inhibitor, a VEGF inhibitor and a PD-1 axis binding antagonist for producing a drug for treating or preventing cancer, the drug being used in combination with a compound 1, a salt thereof or a solvate thereof.

[76] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [75], wherein the compound 1, the salt thereof or

the solvate thereof and the at least one member selected from the group consisting of a KRAS-G12C selective inhibitor, a SHP2 inhibitor, a VEGF inhibitor and a PD-1 axis binding antagonist are used in combination concurrently or separately.

[77] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [76], wherein the compound 1, the salt thereof or the solvate thereof and the at least one member selected from the group consisting of a KRAS-G12C selective inhibitor, a SHP2 inhibitor, a VEGF inhibitor and a PD-1 axis binding antagonist are administered as a combination drug.

[78] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [77], wherein the at least one member selected from the group consisting of a KRAS-G12C selective inhibitor, a SHP2 inhibitor, a VEGF inhibitor and a PD-1 axis binding antagonist is the KRAS-G12C selective inhibitor, the SHP2 inhibitor or the VEGF inhibitor.

[79] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [78], wherein the KRAS-G12C selective inhibitor is at least one member selected from the group consisting of sotorasib, adagrasib, a salt thereof, and a solvate thereof.

[80] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [79], wherein the KRAS-G12C selective inhibitor is adagrasib, a salt thereof or a solvate thereof.

[81] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [79], wherein the KRAS-G12C selective inhibitor is sotorasib, a salt thereof or a solvate thereof.

[82] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [79], [81], wherein the KRAS-G12C selective inhibitor is sotorasib.

[83] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [82], wherein the SHP2 inhibitor is at least one member selected from the group consisting of RMC-4550, TNO155, SHP099, NSC-87877, RMC-4630, GDC-1971, a salt thereof, and a solvate thereof.

[84] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [83], wherein the SHP2 inhibitor is at least one member selected from the group consisting of RMC-4550, TNO155, GDC-1971, a salt thereof, and a solvate thereof.

[85] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], wherein the SHP2 inhibitor is RMC-4550, a salt thereof or a solvate thereof.

[86] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], wherein the SHP2 inhibitor is TNO155, a salt thereof or a solvate thereof.

[87] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], wherein the SHP2 inhibitor is GDC-1971, a salt thereof or a solvate thereof.

[88] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [87], wherein the VEGF inhibitor is bevacizumab.

[89] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [88], wherein the PD-1 axis binding antagonist is an anti-PD-L1 antibody.

[90] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], wherein the at least one member selected from the group consisting of a KRAS-G12C selective inhibitor, a SHP2 inhibitor, a VEGF inhibitor and a PD-1 axis binding antagonist is the KRAS-G12C selective inhibitor.
[91] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [90], wherein the KRAS-G12C selective inhibitor is at least one member selected from the group consisting of sotorasib, adagrasib, a salt thereof, and a solvate thereof.
[92] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [90] or [91], wherein the KRAS-G12C selective inhibitor is adagrasib, a salt thereof or a solvate thereof.
[93] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [90] or [91], wherein the KRAS-G12C selective inhibitor is sotorasib, a salt thereof or a solvate thereof.
[94] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [90], [91] or [93], wherein the KRAS-G12C selective inhibitor is sotorasib.
[95] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], wherein the at least one member selected from the group consisting of a KRAS-G12C selective inhibitor, a SHP2 inhibitor, a VEGF inhibitor and a PD-1 axis binding antagonist is the SHP2 inhibitor.
[96] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [95], wherein the SHP2 inhibitor is at least one member selected from the group consisting of RMC-4550, TNO155, SHP099, NSC-87877, RMC-4630, GDC-1971, a salt thereof, and a solvate thereof.
[97] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [95] or [96], wherein the SHP2 inhibitor is at least one member selected from the group consisting of RMC-4550, TNO155, GDC-1971, a salt thereof, and a solvate thereof.
[98] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [95] to [97], wherein the SHP2 inhibitor is RMC-4550, a salt thereof or a solvate thereof.
[99] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [95] to [97], wherein the SHP2 inhibitor is TNO155, a salt thereof or a solvate thereof.
[100] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [95] to [97], wherein the SHP2 inhibitor is GDC-1971, a salt thereof or a solvate thereof.
[101] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], wherein the at least one member selected from the group consisting of a KRAS-G12C selective inhibitor, a SHP2 inhibitor, a VEGF inhibitor and a PD-1 axis binding antagonist is the VEGF inhibitor.
[102] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [101], wherein the VEGF inhibitor is bevacizumab.
[103] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], wherein the at least one member selected

from the group consisting of a KRAS-G12C selective inhibitor, a SHP2 inhibitor, a VEGF inhibitor and a PD-1 axis binding antagonist is the PD-1 axis binding antagonist.

[104] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [103], wherein the PD-1 axis binding antagonist is an anti-PD-L1 antibody.

[105] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [104], wherein the compound 1, the salt thereof or the solvate thereof is the salt of the compound 1.

[106] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [105], wherein the salt is a sodium salt.

[107] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [78], [83] to [90], wherein the compound 1, the salt thereof or the solvate thereof is a sodium salt of the compound 1, and the at least one member selected from the group consisting of a KRAS-G12C selective inhibitor, a SHP2 inhibitor, a VEGF inhibitor and a PD-1 axis binding antagonist is the KRAS-G12C selective inhibitor.

[108] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [107], wherein the KRAS-G12C selective inhibitor is sotorasib, a salt thereof or a solvate thereof.

[109] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [107], wherein the KRAS-G12C selective inhibitor is adagrasib, a salt thereof or a solvate thereof.

[110] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [85], [88], [89], [95] to [98], wherein the compound 1, the salt thereof or the solvate thereof is a sodium salt of the compound 1, and the at least one member selected from the group consisting of a KRAS-G12C selective inhibitor, a SHP2 inhibitor, a VEGF inhibitor and a PD-1 axis binding antagonist is RMC-4550, a salt thereof or a solvate thereof.

[111] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], [86], [88], [89], [95] to [97], [99], wherein the compound 1, the salt thereof or the solvate thereof is a sodium salt of the compound 1, and the at least one member selected from the group consisting of a KRAS-G12C selective inhibitor, a SHP2 inhibitor, a VEGF inhibitor and a PD-1 axis binding antagonist is TNO155, a salt thereof or a solvate thereof.

[112] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], [87] to [89], [95] to [97], [100], wherein the compound 1, the salt thereof or the solvate thereof is a sodium salt of the compound 1, and the at least one member selected from the group consisting of a KRAS-G12C selective inhibitor, a SHP2 inhibitor, a VEGF inhibitor and a PD-1 axis binding antagonist is GDC-1971, a salt thereof or a solvate thereof.

[113] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], [101], [102], wherein the compound 1, the salt thereof or the solvate thereof is a sodium salt of the compound 1, and the at least one member selected from the group consisting of a KRAS-G12C selective inhibitor, a SHP2 inhibitor, a VEGF inhibitor and a PD-1 axis binding antagonist is bevacizumab.

[114] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], [103], [104], wherein the compound 1, the salt thereof or the solvate thereof is a sodium salt of the compound 1, and the at least one member selected from the group consisting of a KRAS-G12C selective inhibitor, a SHP2 inhibitor, a VEGF inhibitor and a PD-1 axis binding antagonist is an anti-PD-L1 antibody.

[115] The drug, the method, the compound 1, the salt thereof or the solvate thereof for use, the at least one member

selected from the group consisting of a KRAS-G12C selective inhibitor, a SHP2 inhibitor, a VEGF inhibitor and a PD-1 axis binding antagonist for use, or the use according to any of [68] to [104], wherein the compound 1, the salt thereof or the solvate thereof is the compound 1.

[116] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [78], [83] to [90], wherein the compound 1, the salt thereof or the solvate thereof is the compound 1, and the at least one member selected from the group consisting of a KRAS-G12C selective inhibitor, a SHP2 inhibitor, a VEGF inhibitor and a PD-1 axis binding antagonist is the KRAS-G12C selective inhibitor.

[117] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [116], wherein the KRAS-G12C selective inhibitor is sotorasib, a salt thereof or a solvate thereof.

[118] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [116], wherein the KRAS-G12C selective inhibitor is adagrasib, a salt thereof or a solvate thereof.

[119] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [85], [88], [89], [95] to [98], wherein the compound 1, the salt thereof or the solvate thereof is the compound 1, and the at least one member selected from the group consisting of a KRAS-G12C selective inhibitor, a SHP2 inhibitor, a VEGF inhibitor and a PD-1 axis binding antagonist is RMC-4550, a salt thereof or a solvate thereof.

[120] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], [86], [88], [89], [95] to [97], [99], wherein the compound 1, the salt thereof or the solvate thereof is the compound 1, and the at least one member selected from the group consisting of a KRAS-G12C selective inhibitor, a SHP2 inhibitor, a VEGF inhibitor and a PD-1 axis binding antagonist is TNO155, a salt thereof or a solvate thereof.

[121] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [84], [87] to [89], [95] to [97], [100], wherein the compound 1, the salt thereof or the solvate thereof is the compound 1, and the at least one member selected from the group consisting of a KRAS-G12C selective inhibitor, a SHP2 inhibitor, a VEGF inhibitor and a PD-1 axis binding antagonist is GDC-1971, a salt thereof or a solvate thereof.

[122] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], [101], [102], wherein the compound 1, the salt thereof or the solvate thereof is the compound 1, and the at least one member selected from the group consisting of a KRAS-G12C selective inhibitor, a SHP2 inhibitor, a VEGF inhibitor and a PD-1 axis binding antagonist is bevacizumab.

[123] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [89], [103], [104], wherein the compound 1, the salt thereof or the solvate thereof is the compound 1, and the at least one member selected from the group consisting of a KRAS-G12C selective inhibitor, a SHP2 inhibitor, a VEGF inhibitor and a PD-1 axis binding antagonist is an anti-PD-L1 antibody.

[124] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [123], wherein the cancer is solid cancer or blood cancer.

[125] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [68] to [124], wherein the cancer is at least one member selected from the group consisting of lung cancer, esophageal cancer, stomach cancer, colorectal cancer, uterine cancer, ovarian cancer, pancreatic cancer, bladder cancer, thyroid gland cancer, skin cancer, head and neck cancer, leukemia, malignant lymphoma and multiple myeloma.

[126] The drug, the method, the compound 1, the salt thereof or the solvate thereof for use, the at least one member

selected from the group consisting of a KRAS-G12C selective inhibitor, a SHP2 inhibitor, a VEGF inhibitor and a PD-1 axis binding antagonist for use, or the use according to any of [68] to [125], wherein the cancer is a cancer related to abnormality in RAS gene or MAPK/ERK pathway.

[127] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [126], wherein the abnormality in MAPK/ERK pathway is abnormal activation of the MAPK/ERK pathway.

[128] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [126] or [127], wherein the abnormality in MAPK/ERK pathway is abnormal activation due to amplification of a protein in the MAPK/ERK pathway.

[129] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to any of [126] to [128], wherein the abnormality in MAPK/ERK pathway is abnormal activation of the MAPK/ERK pathway due to gene mutation.

[130] The drug, the method, the compound 1, the salt thereof or the solvate thereof for use, the at least one member selected from the group consisting of a KRAS-G12C selective inhibitor, a SHP2 inhibitor, a VEGF inhibitor and a PD-1 axis binding antagonist for use, or the use according to [126], wherein the abnormality in MAPK/ERK pathway is abnormality in RAF gene.

## Advantageous Effect of Invention

The present invention provides a drug that exerts excellent effects on the treatment or prevention of a cancer.

## Brief Description of Drawings

[Figure 1] Figure 1 is an electrophoretic profile showing RAS signal inhibitory activity when a compound 1 was used in combination with sotorasib or adagrasib.

[Figure 2] Figure 2 is an electrophoretic profile showing RAS signal inhibitory activity when a compound 1 was used in combination with TNO155.

[Figure 3] Figure 3 is an electrophoretic profile showing RAS signal inhibitory activity when a compound 1 was used in combination with GDC-1971.

[Figure 4] Figure 4 shows results of analyzing cell proliferation inhibitory activity with an isobologram when the compound 1 was used in combination with sotorasib or adagrasib.

[Figure 5] Figures 5 (a), 5(b) and 5(c) are diagrams showing cell proliferation inhibitory activity against a cell line NCI-H2122 when the compound 1 was used in combination with TNO155.
Figure 5(a) shows results of analyzing cell proliferation inhibitory activity with an isobologram.
Figure 5(b) is a diagram showing proliferation inhibition. Figure 5(c) is a diagram showing EOB.

[Figure 6] Figures 6(a), 6(b) and 6(c) are diagrams showing cell proliferation inhibitory activity against a cell line NCI-H358 when the compound 1 was used in combination with TNO155.
Figure 6(a) shows results of analyzing cell proliferation inhibitory activity with an isobologram.
Figure 6(b) is a diagram showing proliferation inhibition. Figure 6(c) is a diagram showing EOB.

[Figure 7] Figures 7(a), 7(b) and 7(c) are diagrams showing cell proliferation inhibitory activity against a cell line NCI-H2122 when the compound 1 was used in combination with RMC-4550.
Figure 7(a) shows results of analyzing cell proliferation inhibitory activity with an isobologram.
Figure 7(b) is a diagram showing proliferation inhibition. Figure 7(c) is a diagram showing EOB.

[Figure 8] Figures 8(a), 8(b) and 8(c) are diagrams showing cell proliferation inhibitory activity against a cell line NCI-H358 when the compound 1 was used in combination with RMC-4550.
Figure 8(a) shows results of analyzing cell proliferation inhibitory activity with an isobologram.
Figure 8(b) is a diagram showing proliferation inhibition. Figure 8(c) is a diagram showing EOB.

[Figure 9] Figures 9(a), 9(b) and 9(c) are diagrams showing cell proliferation inhibitory activity against a cell line NCI-H1373 when the compound 1 was used in combination with TNO155. Figure 9(a) shows results of analyzing cell proliferation inhibitory activity with an isobologram. The broken line in Figure 9(a) depicts a line that connects the highest treatment concentration 10 $\mu$M of TNO155 and $IC_{50}$ of the compound 1 as a single agent. Figure 9(b) is a diagram showing proliferation inhibition. Figure 9(c) is a diagram showing EOB.

[Figure 10] Figures 10(a), 10(b) and 10(c) are diagrams showing cell proliferation inhibitory activity against a cell line

HCC-1171 when the compound 1 was used in combination with TNO155. Figure 10(a) shows results of analyzing cell proliferation inhibitory activity with an isobologram. Figure 10(b) is a diagram showing proliferation inhibition. Figure 10(c) is a diagram showing EOB.

[Figure 11] Figures 11(a), 11(b) and 11(c) are diagrams showing cell proliferation inhibitory activity against a cell line NCI-H23 when the compound 1 was used in combination with TNO155. Figure 11(a) shows results of analyzing cell proliferation inhibitory activity with an isobologram. Figure 11(b) is a diagram showing proliferation inhibition. Figure 11(c) is a diagram showing EOB.

[Figure 12] Figures 12(a), 12(b) and 12(c) are diagrams showing cell proliferation inhibitory activity against a cell line NCI-H1373 when the compound 1 was used in combination with GDC-1971. Figure 12(a) shows results of analyzing cell proliferation inhibitory activity with an isobologram. Figure 12(b) is a diagram showing proliferation inhibition. Figure 12(c) is a diagram showing EOB.

[Figure 13] Figures 13(a), 13(b) and 13(c) are diagrams showing cell proliferation inhibitory activity against a cell line A-427 when the compound 1 was used in combination with GDC-1971. Figure 13(a) shows results of analyzing cell proliferation inhibitory activity with an isobologram. Figure 13(b) is a diagram showing proliferation inhibition. Figure 13(c) is a diagram showing EOB.

[Figure 14] Figures 14(a), 14(b) and 14(c) are diagrams showing cell proliferation inhibitory activity against a cell line NCI-H1944 when the compound 1 was used in combination with GDC-1971. Figure 14(a) shows results of analyzing cell proliferation inhibitory activity with an isobologram. Figure 14(b) is a diagram showing proliferation inhibition. Figure 14(c) is a diagram showing EOB.

[Figure 15] Figures 15(a), 15(b) and 15(c) are diagrams showing cell proliferation inhibitory activity against a cell line GP2d when the compound 1 was used in combination with GDC-1971. Figure 15(a) shows results of analyzing cell proliferation inhibitory activity with an isobologram. Figure 15(b) is a diagram showing proliferation inhibition. Figure 15(c) is a diagram showing EOB.

[Figure 16] Figures 16(a), 16(b) and 16(c) are diagrams showing cell proliferation inhibitory activity against a cell line HPAC when the compound 1 was used in combination with GDC-1971. Figure 16(a) shows results of analyzing cell proliferation inhibitory activity with an isobologram. Figure 16(b) is a diagram showing proliferation inhibition. Figure 16(c) is a diagram showing EOB.

[Figure 17] Figure 17 is a graph showing antitumor activity and relative body weight change in mice when sodium salt of the compound 1 was used in combination with sotorasib or adagrasib.

[Figure 18] Figure 18 is a graph showing antitumor activity when sodium salt of the compound 1 was used in combination with bevacizumab.

[Figure 19A] Figure 19A is a graph showing antitumor activity against a cell line NCI-H1373 when sodium salt of the compound 1 was used in combination with TNO155.

[Figure 19B] Figure 19B is a graph showing antitumor activity against a cell line NCI-H358 when sodium salt of the compound 1 was used in combination with TNO155.

[Figure 19C] Figure 19C is a graph showing antitumor activity against a cell line NCI-H441 when sodium salt of the compound 1 was used in combination with TNO155.

[Figure 20] Figure 20 is a graph showing antitumor activity when sodium salt of the compound 1 was used in combination with an anti-PD-L1 antibody. Figure 20(a) shows results of using CT26. Figure 20(b) shows results of using mLU6054.

## Description of Embodiments

Embodiments of the present invention will be described. However, the present invention is not limited by the embodiments given below. The drug for treatment or prevention and the method for treatment or prevention of the present invention may be administered or applied to a human. In the present specification, the term "to" which indicates a range comprises values of the upper and lower limits described. For example, the term "A to B" means the range of A or more and B or less. In the present specification, the term "approximately", when used in combination with a numerical value, means the range of +10% and -10% of the numerical value. In the present invention, the term "and/or" comprises every combination in which the terms "and" and "or" are appropriately combined. Specifically, for example, the term "A, B, and/or C" comprises the following seven variations: (i) A, (ii) B, (iii) C, (iv) A and B, (v) A and C, (vi) B and C, and (vii) A, B, and C. In the present specification, the term a "solvate thereof" means a solvate of the compound 1, a solvate of one of the molecular-targeted agents, a solvate of the salt of the compound 1, or a solvate of the salt of one of the molecular-targeted agents.

One embodiment of the present invention provides a drug for treating or preventing cancer, the drug comprising a compound 1, a salt thereof or a solvate thereof and being used in combination with a molecular-targeted agent. Preferably, the molecular-targeted agent is a MAPK/ERK pathway inhibitor. More preferably, the molecular-targeted agent is one selected from the group consisting of a KRAS inhibitor, a SHP2 inhibitor, a VEGF inhibitor and a PD-1 axis binding

antagonist.

[Formula 3]

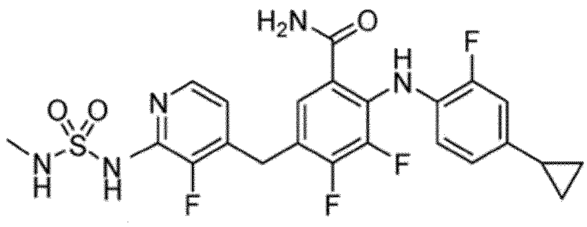

(1)

Another embodiment of the present invention provides a drug for treating or preventing cancer, the drug comprising a molecular-targeted agent and being used in combination with a compound 1, a salt thereof or a solvate thereof. Preferably, the molecular-targeted agent is a MAPK/ERK pathway inhibitor. More preferably, the molecular-targeted agent is one selected from the group consisting of a KRAS inhibitor, a SHP2 inhibitor, a VEGF inhibitor and a PD-1 axis binding antagonist.

One embodiment of the present invention is a drug for treating or preventing cancer, the drug comprising a compound 1, a salt thereof or a solvate thereof and being used in combination with a MAPK/ERK pathway inhibitor.

Another embodiment of the present invention is a drug for treating or preventing cancer, the drug comprising a MAPK/ERK pathway inhibitor and being used in combination with a compound 1, a salt thereof or a solvate thereof.

One embodiment of the present invention is a drug for treating or preventing cancer, the drug comprising a compound 1, a salt thereof or a solvate thereof and being used in combination with a KRAS inhibitor.

Another embodiment of the present invention is a drug for treating or preventing cancer, the drug comprising a KRAS inhibitor and being used in combination with a compound 1, a salt thereof or a solvate thereof.

One embodiment of the present invention is a drug for treating or preventing cancer, the drug comprising a compound 1, a salt thereof or a solvate thereof and being used in combination with a SHP2 inhibitor.

Another embodiment of the present invention is a drug for treating or preventing cancer, the drug comprising a SHP2 inhibitor and being used in combination with a compound 1, a salt thereof or a solvate thereof. The SHP2 inhibitor can be at least one member selected from the group consisting of RMC-4550, TNO155, SHP099, NSC-87877, RMC-4630, GDC-1971, a salt thereof, and a solvate thereof, and can be at least one member selected from the group consisting of RMC-4550, TNO155, GDC-1971, a salt thereof, and a solvate thereof. Preferably, the SHP2 inhibitor is at least one member selected from the group consisting of RMC-4550, TNO155, GDC-1971, a salt thereof, and a solvate thereof, and more preferably, the SHP2 inhibitor is GDC-1971, a salt thereof or a solvate thereof.

One embodiment of the present invention is a drug for treating or preventing cancer, the drug comprising a compound 1, a salt thereof or a solvate thereof and being used in combination with a VEGF inhibitor.

Another embodiment of the present invention is a drug for treating or preventing cancer, the drug comprising a VEGF inhibitor and being used in combination with a compound 1, a salt thereof or a solvate thereof. The VEGF inhibitor can be at least one member selected from the group consisting of sorafenib, pazopanib, sunitinib, axitinib, regorafenib, a salt thereof, and a solvate thereof, and an anti-VEGF antibody, can be an anti-VEGF antibody, and can be bevacizumab, ramucirumab, or aflibercept. Preferably, the VEGF inhibitor is the anti-VEGF antibody, and more preferably, the VEGF inhibitor is bevacizumab.

Another embodiment of the present invention is a drug for treating or preventing cancer, the drug comprising a PD-1 axis binding antagonist and being used in combination with a compound 1, a salt thereof or a solvate thereof. The PD-1 axis binding antagonist can be at least one member selected from the group consisting of a PD-1 binding antagonist, a PD-L1 binding antagonist and a PD-L2 binding antagonist. The PD-1 binding antagonist can be an anti-PD-1 antibody, and can be at least one member selected from the group consisting of MDX-1106 (nivolumab), MK-3475 (pembrolizumab, lambrolizumab), CT-011 (pidilizumab), PDR001, REGN2810, BGBA317, SHR-1210, AMP-514 (MEDI0680) and AMP-224. The PD-L1 binding antagonist can be a small molecule, can be a small molecule having a molecular weight

of 2000 g/mol or less, can be INCB086550, a salt thereof or a solvate thereof, can be an anti-PD-L1 antibody, and can be at least one member selected from the group consisting of YW243.55.S70, atezolizumab, MPDL3280A, MDX-1105, avelumab, and MEDI4736 (durvalumab). The PD-L2 binding antagonist can be an anti-PD-L2 antibody, and can be immunoadhesin. Preferably, the PD-1 axis binding antagonist is a PD-1 binding antagonist or a PD-L1 binding antagonist, and more preferably, the PD-1 axis binding antagonist is a PD-L1 binding antagonist. Preferably, the PD-1 binding antagonist is an anti-PD-1 antibody, and more preferably, the PD-1 binding antagonist is MDX-1106 (nivolumab) or MK-3475 (pembrolizumab). Preferably, the PD-L1 binding antagonist is an anti-PD-L1 antibody, and more preferably, the PD-L1 binding antagonist is atezolizumab.

One embodiment of the present invention is a drug for treating or preventing cancer, the drug comprising a compound 1, a salt thereof or a solvate thereof and being used in combination with a KRAS-G12C selective inhibitor.

Another embodiment of the present invention is a drug for treating or preventing cancer, the drug comprising a KRAS-G12C selective inhibitor and being used in combination with a compound 1, a salt thereof or a solvate thereof. The KRAS-G12C selective inhibitor can be at least one member selected from the group consisting of sotorasib, adagrasib, a salt thereof, and a solvate thereof, can be sotorasib or a salt thereof or a solvate thereof, and can be adagrasib or a salt thereof or a solvate thereof. Preferably, the KRAS-G12C selective inhibitor is at least one member selected from the group consisting of sotorasib, adagrasib, a salt thereof, and a solvate thereof.

The compound 1, the salt thereof or the solvate thereof and the molecular-targeted agent can be provided as a kit. A further alternative embodiment of the present invention may provide a kit comprising: a formulation containing a compound 1, a salt thereof or a solvate thereof; and a molecular-targeted agent. The drug may be provided as a kit. Another embodiment of the of the present invention can be a kit comprising the drug.

The "use in combination" means that two or more ingredients are used in combination. For example, the use in combination of the compound 1, the salt thereof or the solvate thereof (hereinafter, also referred to as the "first ingredient") and the molecular-targeted agent (hereinafter, also referred to as the "second ingredient") comprises an "aspect in which the first ingredient and the second ingredient are administered as a single formulation comprising these ingredients" (i.e., an aspect in which the first ingredient and the second ingredient are used in combination as a combination drug) and an "aspect in which the first ingredient and the second ingredient are administered as different formulations concurrently or separately". In the latter aspect, the formulation containing the first ingredient may be administered first, or the formulation containing the second ingredient may be administered first. The latter aspect can be any of an "aspect in which the first ingredient and the second ingredient are separately formulated and the formulations are concurrently administered through the same administration route", an "aspect in which the first ingredient and the second ingredient are separately formulated and the formulations are separately administered in a staggered manner through the same administration route", an "aspect in which the first ingredient and the second ingredient are separately formulated and the formulations are concurrently administered through different administration routes (administered from different sites of the same patient)", and an "aspect in which the first ingredient and the second ingredient are separately formulated and the formulations are separately administered in a staggered manner through different administration routes". In the "aspect in which the first ingredient and the second ingredient are separately formulated and the formulations are concurrently administered through the same administration route", both the formulations may be mixed immediately before administration. The term "separately" means that a certain formulation is administered before or after administration of another formulation.

In other words, the "use in combination" may be regarded as a use method of allowing one ingredient to exist in the body of a patient in a state where another ingredient exists in the body of the patient. Specifically, an aspect is preferred in which the first ingredient and the second ingredient are administered such that these ingredients exist concurrently in the body, for example, blood, of a patient. An aspect is preferred in which a certain drug is administered to a patient concurrently with another drug or within 48 hours after administration of another drug.

The compound 1 is 2-(4-cyclopropyl-2-fluoro-anilino)-3,4-difluoro-5-[[3-fluoro-2-(methylsulfamoylamino)-4-pyridyl]methyl]benzamide (CAS No: 2677856-11-8).

The first ingredient may be the compound 1, a salt of the compound 1, a solvate of the salt of the compound 1, or a solvate of the compound 1.

Examples of the salt of the compound 1 comprise pharmaceutically acceptable salts and can specifically comprise: salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salt; and salts with amino acids such as arginine. These salts are produced, for example, by contacting the compound 1 with an acid or a base. In the present specification, a solvate is not particularly limited as long as the compound 1 forms a single molecular population together with a solvent, and is a solvate formed by a solvent which is acceptable for ingestion concomitantly with administration of a drug. Examples thereof can include not only solvates with a single solvent such as hydrate, alcoholic hydrate (ethanol hydrate, methanol hydrate, 1-propanol hydrate, 2-propanol hydrate, and the like),

dimethyl sulfoxide, but also those obtained by forming solvates by a plurality of solvents with respect to 1 molecules of the compound 1, or those obtained by forming solvates by a plurality of kinds of solvents with respect to 1 molecules of the compound 1. If the solvent is water, the solvate is called hydrate. These solvates are produced, for example, by contacting the compound 1 with a solvent, and these solvates of salts are produced, for example, by contacting a salt of the compound 1 with a solvent. The salt of the compound 1 and the solvate of the compound 1 or the salt of the compound 1 are all active as well as the compound 1 (free form). The salt of the compound 1 is preferably sodium salt. The compound 1 is capable of forming, for example, sodium salt represented by the formula (2) given below. The molecular-targeted agent mentioned later can also be in the form of a salt exemplified as the salt of the compound 1 or a solvate. The salt or the solvate of the molecular-targeted agent can be obtained by a production method exemplified as the method for producing the salt or the solvate of the compound 1.

[Formula 4]

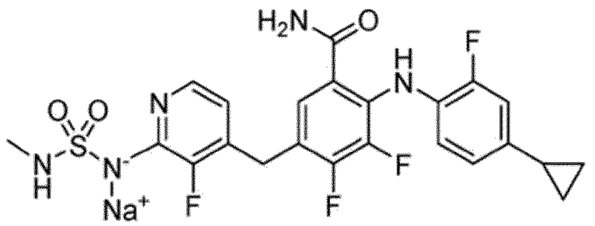

(2)

The first ingredient may have crystal polymorphs and may be a single material of any crystal form or any mixture of crystal forms. The first ingredient also comprises an amorphous material.

The dose of the first ingredient is preferably 0.0001 to 50 mg per kg of the body weight of a recipient per day (0.0001 to 50 mg/kg/day), more preferably 0.001 to 2 mg/kg/day, further preferably 0.002 to 0.2 mg/kg/day. When the dose of the first ingredient falls within these ranges, therapeutic or prophylactic effects on a cancer are more enhanced. The frequency of administration of the first ingredient can be, for example, once a day, twice a day, or three times a day and is more preferably once a day.

The first ingredient can be produced in accordance with, for example, a method described in International Publication No. WO 2021/149776.

In the present specification, the "molecular-targeted agent" means an agent that has antitumor effects and specifically inhibits a particular protein. In the present specification, the molecular-targeted agent does not comprise the compound 1, the salt thereof or the solvate of the compound or the salt. Examples of the molecular-targeted agent comprise a MAPK/ERK pathway inhibitor, which is applicable to a drug for treating or preventing cancer. In the present specification, the term "MAPK/ERK- pathway inhibitor" refers to an inhibitor of targets that involve MAPK/ERK signals, including inhibitors of growth factors and their receptors, and kinase inhibitors. For example, the MAPK/ERK- pathway inhibitor may include an EGFR inhibitor, a VEGF inhibitor, a SHP2 inhibitor, a KRAS inhibitor, a NRAS inhibitor, a HRAS inhibitor, a KRAS-GTP inhibitor, a NRAS-GTP inhibitor, a HRAS-GTP inhibitor, a KRAS-G12C selective inhibitor, an ERK inhibitor, an AKT inhibitor, and a MEK inhibitor. The molecular-targeted agent can be at least one member selected from the group consisting of a KRAS inhibitor, a SHP2 inhibitor, a VEGF inhibitor and a PD-1 axis binding antagonist, and may be a KRAS inhibitor, a SHP2 inhibitor, a VEGF inhibitor or a PD-1 axis binding antagonist. The KRAS inhibitor can be a KRAS-G12C selective inhibitor. In this case, the molecular-targeted agent can be at least one member selected from the group consisting of a KRAS-G12C selective inhibitor, a SHP2 inhibitor, a VEGF inhibitor and a PD-1 axis binding antagonist, and may be a KRAS-G12C selective inhibitor, a SHP2 inhibitor, a VEGF inhibitor or a PD-1 axis binding antagonist. Preferably, the molecular-targeted agent is a KRAS-G12C selective inhibitor. The KRAS-G12C selective inhibitor can be at least one member selected from the group consisting of sotorasib, adagrasib, a salt thereof, and a solvate thereof, can be sotorasib or a salt thereof or a solvate thereof, and can be adagrasib or a salt thereof or a solvate thereof. Preferably, the KRAS-G12C selective inhibitor is at least one member selected from the group consisting of sotorasib, adagrasib, a salt thereof, and a solvate thereof. Preferably, the molecular-targeted agent is a SHP2 inhibitor. The SHP2 inhibitor can be at least one member selected from the group consisting of RMC-4550, TNO155, SHP099, NSC-87877, RMC-4630, GDC-1971, a salt thereof, and a solvate thereof, and can be at least one member selected from the group consisting of RMC-4550, TNO155, GDC-1971, a salt thereof, and a solvate thereof. Preferably, the SHP2 inhibitor is at least one member selected from the group consisting of RMC-4550, TNO155, GDC-1971, a salt thereof, and a solvate thereof, and more preferably, the SHP2 inhibitor is GDC-1971, salt thereof or solvate thereof. The VEGF inhibitor can be at least one member selected from the group consisting of sorafenib, pazopanib, sunitinib, axitinib, regorafenib, a salt thereof, and a solvate thereof, and an anti-VEGF antibody, can be an anti-VEGF antibody, and can be bevacizumab, ramucirumab, or aflibercept. Preferably, the VEGF inhibitor is the anti-VEGF antibody, and more preferably, the VEGF inhibitor is bevacizumab. Preferably, the PD-1 axis binding antagonist is a PD-1 binding antagonist or a PD-L1 binding antagonist, and more preferably, the PD-1 axis

binding antagonist is a PD-L1 binding antagonist. Preferably, the PD-1 binding antagonist is an anti-PD-1 antibody, and more preferably, the PD-1 binding antagonist is MDX-1106 (nivolumab) or MK-3475 (pembrolizumab). Preferably, the PD-L1 binding antagonist is an anti-PD-L1 antibody, and more preferably, the PD-L1 binding antagonist is atezolizumab.

The molecular-targeted agent may be obtained from a commercial supplier or may be produced in accordance with a method known in the art.

Sotorasib (CAS No: 2296729-00-3; 4-((S)-4-acryloyl-2-methylpiperazin-1-yl)-6-fluoro-7-(2-fluoro-6-hydroxyphenyl)-1-(2-isopropyl-4-methylpyridin-3-yl)pyrido[2,3-d]pyrimidin-2(1H)-one) is a compound represented by the formula (3) given below. Sotorasib is used as AMG510 or Lumakras.

[Formula 5]

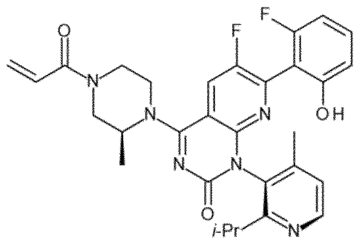

(3)

Adagrasib (CAS No: 2326521-71-3; MRTX849 or 2-((S)-4-(7-(8-chloronaphthalen-1-yl)-2-(((S)-1-methylpyrrolidin-2-yl)methoxy)-5,6,7,8-tetrahydropyrido[3,4-d]pyrimidin-4-yl)-1-(2-fluoroacryloyl)piperazin-2-yl)acetonitrile) is a compound represented by the following formula (4).

[Formula 6]

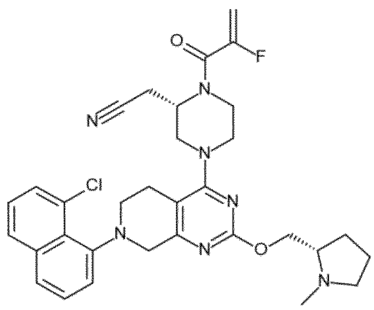

(4)

RMC-4550 (CAS No: 2172651-73-7; 3-[(3S,4S)-4-amino-3-methyl-2-oxa-8-azaspiro[4.5]dec-8-yl]-6-(2,3-dichlorophenyl)-5-methyl-2-pyrazinemethanol) is a compound represented by the following formula (5).

[Formula 7]

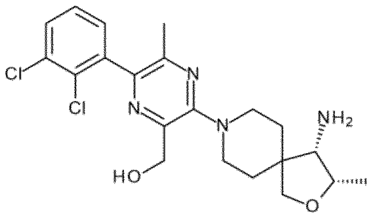

(5)

TNO155 (CAS No: 1801765-04-7; (3S,4S)-8-(6-amino-5-((2-amino-3-chloropyridin-4-yl)thio)pyrazin-2-yl)-3-methyl-2-oxa-8-azaspiro[4.5]decan-4-amine) is a compound represented by the following formula (6).

[Formula 8]

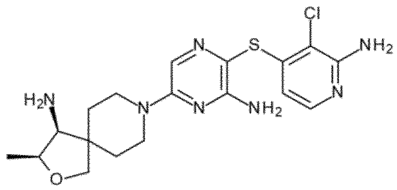

(6)

SHP099 (CAS No: 1801747-42-1; 6-(4-amino-4-methyl-1-piperidinyl)-3-(2,3-dichlorophenyl)-2-pyrazinamine) is a compound represented by the following formula (7).

[Formula 9]

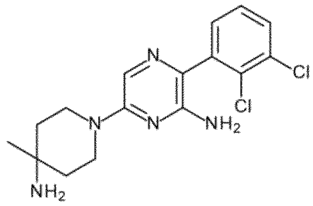

(7)

NSC-87877 (CAS No: 56990-57-9; 8-hydroxy-7-[2-(6-sulfo-2-naphthalenyl)diazenyl]-5-quinolinesulfonic acid) is a compound represented by the following formula (8).

[Formula 10]

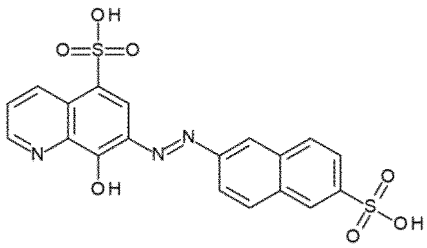

(8)

RMC-4630 is a compound described in Non Patent Literature 1.

Sorafenib (CAS No: 284461-73-0; 4-[4-[[4-chloro-3-(trifluoromethyl)phenyl]carbamoylamino]phenoxy]-N-methyl-pyridine-2-carboxamide) is a compound represented by the formula (9) given below. Sorafenib is used as sorafenib tosylate or Nexavar.

[Formula 11]

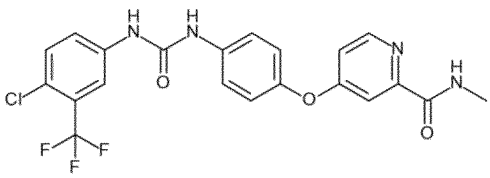

(9)

Pazopanib (CAS No: 444731-52-6; 5-[[4-[(2,3-dimethylindazol-6-yl)-methyl-amino]pyrimidin-2-yl]amino]-2-methyl-benzenesulfonamide) is a compound represented by the formula (10) given below. Pazopanib is used as pazopanib hydrochloride or Votrient.

[Formula 12]

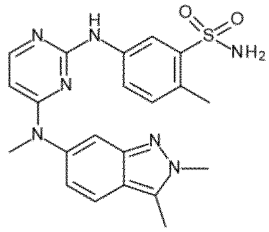

(10)

Sunitinib (CAS No: 557795-19-4; N-[2-(diethylamino)ethyl]-5-[(Z)-(5-fluoro-2-oxo-indolin-3-ylidene) methyl]-2,4-dimethyl-1H-pyrrole-3-carboxamide) is a compound represented by the formula (11) given below. Sunitinib is used as sunitinib malate or Sutent.

[Formula 13]

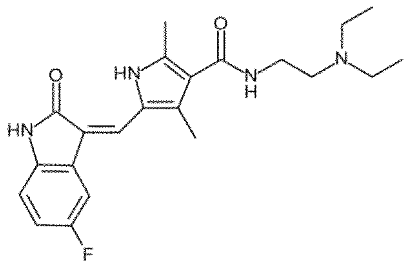

(11)

Axitinib (CAS No: 319460-85-0; N-methyl-2-[[3-[(E)-2-(2-pyridyl)vinyl]-1H-indazol-6-yl]sulfanyl]benzamide) is a compound represented by the formula (12) given below. Axitinib is used as Inlyta.

[Formula 14]

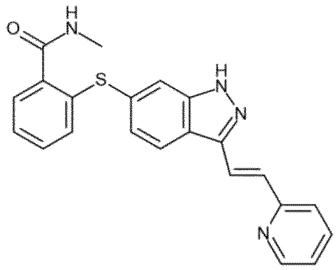

(12)

Regorafenib (CAS No: 755037-03-7; 4-[4-[[4-chloro-3-(trifluoromethyl)phenyl]carbamoylamino]-3-fluoro-phenoxy]-N-methyl-pyridine-2-carboxamide) is a compound represented by the formula (13) given below. Regorafenib is used as regorafenib hydrate or Stivarga.

[Formula 15]

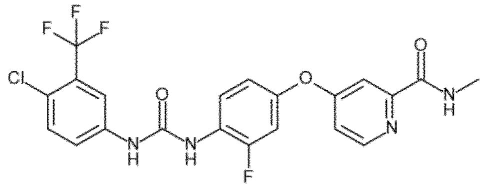

(13)

Bevacizumab is an antibody designated as bevacizumab under Japanese Accepted Names for Pharmaceuticals (abbreviated as JAN), represented by CAS Registry No. 216974-75-3 or INN name bevacizumab. Bevacizumab is also a recombinant humanized monoclonal antibody and consists of complementarity determining regions of a mouse anti-human vascular endothelial growth factor (VEGF) monoclonal antibody, human framework regions and human IgG1

constant regions. Bevacizumab is produced by Chinese hamster ovary cells. Bevacizumab is a glycoprotein (molecular weight: approximately 149,000) composed of two H chains (γ1 chains) each consisting of 453 amino acid residues, and two L chains (κ chains) each consisting of 214 amino acid residues. "Bevacizumab" also comprises biosimilars of bevacizumab.

GDC-1971 (CAS No: 2377352-49-1; (R)-1'-(3-(3,4-dihydro-1,5-naphtyridin-1(2H)-yl)-1H-pyrazolo[3,4-b]pyradin-6-yl)-3H-spiro[benzofuran-2,4'-piperidin]-3-amine) is a compound represented by the following formula (14).

[Formula 16]

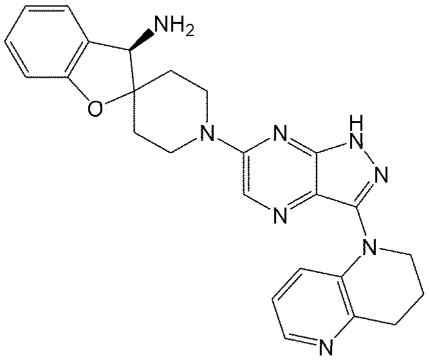

(14)

The dose of the molecular-targeted agent is preferably 0.005 to 300 mg per kg of the body weight of a recipient per day (0.005 to 300 mg/kg/day), more preferably 0.01 to 250 mg/kg/day, further preferably 0.02 to 200 mg/kg/day. When the dose of the molecular-targeted agent falls within these ranges, therapeutic or prophylactic effects on a cancer are more enhanced. The frequency of administration of the molecular-targeted agent can be, for example, once or more a week and can be twice a week.

The doses of the first ingredient and the second ingredient are preferably 0.0001 to 50 mg/kg/day of the first ingredient and 0.005 to 300 mg/kg/day of the second ingredient, more preferably 0.001 to 2 mg/kg/day of the first ingredient and 0.01 to 250 mg/kg/day of the second ingredient, further preferably 0.002 to 0.2 mg/kg/day of the first ingredient and 0.02 to 200 mg/kg/day of the second ingredient (the term "/kg" means per kg of the body weight of a recipient). When the doses of the first ingredient and the second ingredient fall within these ranges, therapeutic or prophylactic effects on a cancer are more enhanced. The dosage and frequency of administration can be adjusted accordingly while monitoring blood drug level parameters such as AUCs and Cmax and the patient's status. Appropriate dosages and frequencies of administration can also be referred to, for example, the package inserts of the molecular-targeted agents.

In the present invention, examples of the administration method comprise administration through an oral, rectal, parenteral (intravenous, intramuscular, subcutaneous, or transdermal absorption), intracisternal, intravaginal, intraperitoneal, intravesical, or local (injection, drop infusion, powders, ointments, gels or creams) route, and inhalation (oral or nasal spray). Examples of the dosage form therefor comprise tablets, capsules, granules, powders, pills, aqueous and nonaqueous oral solutions and suspensions, and parenteral solutions filled in containers adapted to segmentation into individual doses. Alternatively, the dosage form may be adapted to various administration methods comprising controlled-release formulations, such as subcutaneous transplantation. The administration methods are equally applicable to the first and second ingredients. The first and second ingredients can be used as a drug and may be administered in a formulation or as a combination drug.

The first ingredient can be administered by, for example, any of the administration methods described above, and the second ingredient can be administered by, for example, the same or different administration method as or from that of the first ingredient. The interval of administration between the first ingredient and the second ingredient can be, for example, a 0-day to 14-day interval, a 0-day to 10-day interval, or a 0-day to 7-day interval. The interval of administration can be determined by using, for example, AUC, Cmax, Tmax, elimination half-life, or the health condition of a subject as an index. For example, the first ingredient and the second ingredient can be administered orally (tablets, capsules, etc.) and through drop infusion, respectively. For example, the first ingredient and the second ingredient can both be administered orally (tablets, capsules, etc.). For example, the first ingredient and the second ingredient can be administered through drop infusion and orally (tablets, capsules, etc.), respectively. For example, the first ingredient and the second ingredient can both be administered through drop infusion. In such a case, the first ingredient and the second ingredient can be administered at an arbitrary interval such as twice a day, once a day, once a week, or once every two weeks. More specifically, the first ingredient and the second ingredient can both be administered once a day. In this case, these ingredients can be administered before a meal, between meals, or after a meal. The term "before a meal", "between meals", or "after a meal" can be before, between, or after any of breakfast, lunch, dinner, late-night meal, and between-meal eating. Provided that the interval of administration between the first ingredient and the second ingredient is within 24 hours, it may mean that both the ingredients are administered once a day. If necessary, the first ingredient and the second ingredient may be administered twice a day and once a day, respectively, administered once a day and once a day, respectively, administered once a day and twice a day, respectively, administered once a day and once every two days, respectively, administered once a day and once every three days, respectively, administered once a day and once every seven days, respectively, or administered twice a day and once every seven days, respectively. If necessary, only the interval of administration of the second ingredient may be increased or decreased, and only the interval of administration of the first ingredient may be increased or decreased based on AUC, Cmax, Tmax, elimination half-life of the compound 1 and/or the molecular-targeted agent, or health status of a subject. The administration of the second ingredient may be started on the start day of administration of the first ingredient, or the start day of administration of the first ingredient and the start day of administration of the second ingredient may be different. As for an administration period, one course involves 7 days, and the total number of courses can be set to one or more courses and may be two or more courses. The respective courses may be continuously performed, or a drug holiday may be established. A drug holiday may be established during a course. If necessary, during courses, only the first ingredient may be continuously administered with cessation of the second ingredient, while the second ingredient may be continuously administered with cessation of only the first ingredient. The first ingredient and the second ingredient may be contained in the same tablet, capsule, or the like.

A pharmaceutical formulation can be prepared by introducing a pharmaceutically acceptable carrier into an active ingredient exemplified by the compound 1, a salt thereof or a solvate thereof, or a molecular-targeted agent of the present invention and formulating it in a known manner. Excipients, binders, lubricants, colorants, flavoring agents, and, if necessary, stabilizers, emulsifiers, absorption promoters, surfactants, pH adjusters, preservatives, antioxidants, and the like, which are usually used for formulation, can be used, and generally, components used as raw materials for pharmaceutical formulations can be blended and formulated by a conventional method. In the formulation, an active ingredient used in a drug can be processed into an optimum shape or property, that is, a dosage form, according to the method of use and the purpose of use by a known method. Typically used dosage forms include, but are not limited to, liquid pharmaceutical formulations (liquid formulations) such as injectables, suspensions, emulsions, and eye drops, and solid pharmaceutical formulations (solid formulations) such as tablets, powder, fine granules, granules, coated tablets, capsules, dry syrups, lozenges, and suppositories. For example, formulations comprising an active ingredient exemplified by the compound 1, a salt thereof or a solvate thereof, or a molecular-targeted agent, can be used as drugs of the present invention, respectively.

The drug for treatment or prevention of the present invention may be used by mixing any or both of the first ingredient and the second ingredient with a pharmaceutically acceptable additive. Those skilled in the art can appropriately select a well-known additive as the pharmaceutically acceptable additive and appropriately use a plurality of additives, if necessary. The additive is appropriately selected depending on an oral agent or an injection and may be, for example, a mixture of water, ethanol, physiological saline, liquid paraffin, a surfactant, and sucrose or may be the resulting mixture filled in a capsule.

Cancer can be classified as either solid cancers or blood cancers. Both types are composed of abnormal cells that multiply uncontrollably. Solid cancers create a single mass or many masses, whereas blood cancers circulate throughout the body via the bloodstream. Examples of the cancer comprise solid cancer and blood cancer. The cancer can be, for example, at least one member selected from the group consisting of lung cancer, esophageal cancer, stomach cancer, colorectal cancer, uterine cancer, ovarian cancer, pancreatic cancer, bladder cancer, thyroid gland cancer, skin cancer, head and neck cancer, leukemia, malignant lymphoma and multiple myeloma. Preferably, the cancer is at least one member selected from the group consisting of lung cancer, ovarian cancer, colorectal cancer, and pancreatic cancer.

The cancer can be a cancer related to abnormality in RAS gene or abnormality in MAPK/ERK pathway. Examples of the abnormality in MAPK/ERK pathway comprise abnormal activation of the MAPK/ERK pathway and comprise abnormal activation of the MAPK/ERK pathway due to the amplification of a protein present in the MAPK/ERK pathway, abnormal activation of the MAPK/ERK pathway due to mutation in a gene encoding a protein present in the MAPK/ERK pathway, and abnormal activation of the MAPK/ERK pathway due to abnormality in RAF gene.

"Abnormality" refers to a state in which various chromosomes, genes, proteins, and signaling cascades in a cell are distant from normal work and control, usually triggered by these constitutive activations.

"Abnormal activation" refers to the state in which various chromosomes, genes, proteins, and signal cascades in cells are more activated than in normal cells, for example, mutations of genes can be one of the causes (Cancer Metastasis Rev, 2013, 32, 147-162), and cellular activation can be measured by, for example, LC/MS-MS-based assay to directly and quantitatively determine engagement of mutant in a cellular setting (Cancer Discov., 2016, 6, 316-329).

"Amplification of a protein" refers to the synthesis and amplification of proteins in living cells, such as the amplification of proteins involved in a MAPK/ERK pathway (e.g., KRAS, NRAS, HRAS, KRAS-GTP, NRAS-GTP, HRAS-GTP, ERK, AKT, MEK, pERK, pAKT, and pMEK). Amplification of the proteins can be confirmed by measuring the protein by Western blotting, immunohistochemical staining (IHC) or other methods and comparing it with the protein in normal cells (Nat. Rev. Drug Discov. 19, 533-552 (2020)., Molecular Cytogenetics, 2015, 8: 103).

"Gene mutation" means that a particular base in a nucleotide sequence is different from the base in the corresponding wild-type nucleotide sequence.

The "treatment of a cancer" according to the present invention means decrease in the number of cancer cells in an individual, the suppression of proliferation of cancer cells, decrease in tumor volume, decrease in tumor weight, the suppression of metastasis of cancer cells, or improvement in various symptoms caused by the cancer. The "prevention of a cancer" according to the present invention means the prevention of increase in the number of cancer cells ascribable to the re-proliferation of cancer cells whose number has been decreased, the prevention of re-proliferation of cancer cells whose proliferation has been suppressed, or the prevention of re-increase in decreased volume or weight of tumor.

Examples

The contents of the present invention will be further described with reference to Examples given below. However, the present invention is not limited by the contents thereof. All compounds and reagents were obtained from commercial suppliers or synthesized by use of methods known in the art. In the present Examples, each molecular-targeted agent is also referred to as a combined-use compound. In the present Examples, the "sodium salt of the compound 1" is a compound represented by the formula (2). In Figure 17, the "sodium salt of the compound 1" is abbreviated as "compound 1".

Compound 1 (2-(4-cyclopropyl-2-fluoro-anilino)-3,4-difluoro-5-[[3-fluoro-2-(methylsulfamoylamino)-4-pyridyl] methyl]benzamide) and sodium salt thereof were produced in accordance with the methods described in International Publication No. WO 2021/149776. The molecular-targeted agents (sotorasib, adagrasib, TNO155, RMC-4550, GDC-1971 and bevacizumab) were supplied from commercial suppliers or manufacturers. The anti-PD-L1 antibody used was prepared by changing constant regions of a commercially available anti-PD-L1 antibody (BioXCell, clone: 10F.9G2) to mouse ones.

<Pharmacological Test Examples>

(Example 1: *In vitro* RAS signal inhibitory activity evaluation)

Single-agent treatment with the compound 1, sotorasib, adagrasib, TNO155 or GDC-1971, combined treatment with the compound 1 and sotorasib, combined treatment with the compound 1 and adagrasib, combined treatment with the compound 1 and TNO155, and combined treatment with the compound 1 and GDC-1971 were evaluated for their influence on human cancer cells. More specifically, the influence of the single-agent treatment or the combined treatment mentioned above on the phosphorylation of MEK and ERK and on the binding between KRAS and GTP within human cancer cells was evaluated by Western blot.

The human cancer cells used were KRAS mutation-positive human lung cancer cell lines NCI-H2122 (KRAS-G12C mutation), NCI-H1373 (KRAS-G12C mutation), NCI-H441 (KRAS-G12V mutation) and HPAC (KRAS-G12D) (all, ATCC). These cells were inoculated to a three-dimensional culture plate PrimeSurface (Sumitomo Bakelite Co., Ltd.) containing a medium. The media used are shown in Table 2. The plate inoculated with the cells was cultured at 37°C in a 5% carbon dioxide incubator for 24 hours. Then, 3 nmol/L, 10 nmol/L, 30 nmol/L or 300 nmol/L compound 1, 200 nmol/L sotorasib, 1000 nmol/L adagrasib, 1000 nmol/L TNO155, 100 nmol/L GDC-1971, 300 nmol/L compound 1 and 200 nmol/L sotorasib, 300 nmol/L compound 1 and 1000 nmol/L adagrasib, 10 nmol/L or 30 nmol/L compound 1 and 1000 nmol/L TNO155, or 3 nmol/L or 10 nmol/L compound 1 and 100 nmol/L GDC-1971 were added to the cells (all the concentrations described here are final concentrations). The plate thus subjected to single-agent treatment or combined treatment was cultured at 37°C in a 5% carbon dioxide incubator. After a lapse of 4 hours, 24 hours or 72 hours, the cells were recovered. The recovered cells were lysed with Cell Lysis Buffer (Cell Signaling Technology, Inc.) containing protease and phosphatase inhibitors (Thermo Fisher Scientific Inc.) to obtain a protein solution.

RAS-GTP, an activated form of RAS, was recovered from a portion of the obtained protein solution using Active Ras Pull-Down and Detection Kit (Thermo Fisher Scientific Inc.). SDS-PAGE was carried out using the obtained RAS-GTP concentrated solution and the protein solution, and migrated proteins were transferred to a PVDF membrane using Trans-Blot Turbo Transfer System (Bio-Rad Laboratories, Inc.). After blocking, the PVDF membrane was reacted with a primary antibody and a secondary antibody in this order. The chemiluminescence of protein bands was caused with Chemi-lumi One Super (Nacalai Tesque, Inc.). The bands were detected with ChemiDoc Touch Imaging System (Bio-Rad Laboratories, Inc.).

Table 1 shows the primary antibody (reacted overnight at 4°C) and the secondary antibody (reacted at room temperature for 45 minutes to 60 minutes) used, and the dilution ratio of each antibody. Figures 1, 2 and 3 show results of Western blotting performed to evaluate RAS signal inhibitory activity. "KRAS-GTP" depicts KRAS bound with GTP. "pMEK" and "pERK" depict phosphorylated MEK and ERK, respectively. Figure 1 revealed inhibited pMEK and pERK after 4 hours and 24 hours from single-agent treatment with the compound 1. Although inhibited KRAS-GTP, pMEK and pERK

were found after 4 hours from single-agent treatment with sotorasib or adagrasib, the inhibition of pERK was attenuated after 24 hours from the treatment. On the other hand, combined treatment with the compound 1 and sotorasib or adagrasib was found to inhibit pERK even after 24 hours from the combined treatment. Figures 2 and 3 revealed that although inhibited pMEK, pERK, DUSP4 and DUSP6 were found by single-agent treatment with the compound 1, TNO155 or GDC-1971, the inhibition of pMEK and pERK tended to be attenuated as time passed. The degrees of inhibition of pMEK, pERK, DUSP4 and DUSP6 by combined treatment with the compound 1 and TNO155 or GDC-1971 were stronger than by single-agent treatment, indicating that the attenuation of inhibition by lapse of time was less likely to occur.

[Table 1]

| Antibody | Manufacturer | Catalog No. | Dilution ratio |
|---|---|---|---|
| Monoclonal Anti-KRAS antibody | Sigma-Aldrich | WH0003845M1 | 1/500 |
| Phospho-MEK1/2 (Ser217/221) Antibody | Cell Signaling Technology | 9121 | 1/1000 |
| MEK1/2 Antibody | Cell Signaling Technology | 9122 | 1/1000 |
| Phospho-p44/42 MAPK (Erk1/2) (Thr202/Tyr204) Antibody | Cell Signaling Technology | 9101 | 1/500 |
| p44/42 MAPK (Erk1/2) Antibody | Cell Signaling Technology | 9102 | 1/2000 |
| DUSP4/MKP2 (D9A5) Rabbit mAb | Cell Signaling Technology | 5149 | 1/1000 |
| DUSP6/MKP3 Antibody | Cell Signaling Technology | 39441 | 1/1000 |
| GAPDH (14C10) Rabbit mAb | Cell Signaling Technology | 2118 | 1/8000 |
| Anti-rabbit IgG, HRP-linked Antibody | Cell Signaling Technology | 7074 | 1/25000 - 1/10000 |
| Anti-mouse IgG, HRP-linked Antibody | Cell Signaling Technology | 7076 | 1/10000 |

(Example 2: Cell proliferation inhibitory activity evaluation)

Single-agent treatment with the compound 1, sotorasib, adagrasib, TNO155, RMC-4550 or GDC-1971 and combined treatment with the compound 1 and any of these molecular-targeted agents (combined-use compounds) were evaluated for their influence on the proliferation of human cancer cells by the ATP level measurement of live cells. These combined-use compounds and the compound 1 were each prepared into dilution series with dimethyl sulfoxide (DMSO). Dimethyl sulfoxide alone or each combined-use compound and/or the compound 1 was dispensed into wells of a U-bottom 96-well plate or a U-bottom 384-well plate using a liquid dispenser Echo (LABCYTE). A KRAS mutation-positive human cancer cell line NCI-H2122, NCI-H358, MIA PaCa-2 (all, ATCC), NCI-H1373, NCI-H23, NCI-H1944, A-427, HPAC (all, ATCC), HCC-1171 (KCLB) or GP2d (ECACC) described in Table 2 was inoculated to the wells supplemented with dimethyl sulfoxide alone (dimethyl sulfoxide treatment well), or the wells supplemented with the combined-use compound and/or the compound 1 (compound treatment well). The plate inoculated with the cancer cells was cultured at 37°C in a 5% carbon dioxide incubator. After a lapse of a predetermined period from the start of culture, CellTitor Glo 2.0 (Promega Corp.) was added to the plate, and an ATP level was measured using EnVision plate reader (Perkin Elmer, Inc.). Cell proliferation inhibitory activity (proliferation inhibition (%)) was calculated according to the expression (1) given below. Here, the predetermined period was 6 days when the combined-use compound used was TNO155 and the cell lines used were NCI-H1373, HCC-1171 and NCI-H23 and 7 days in the other cases.

[Table 2]

| Cell name | KRAS mutation | Cancer type | Medium |
|---|---|---|---|
| NCI-H2122 | G12C | Lung cancer | RPMI-1640 medium supplemented with 10% fetal bovine serum, 2.5 g/L D-(+)-glucose solution, 10 mmol/L HEPES (all, Sigma-Aldrich), and 1 mmol/L sodium pyruvate (gibco) |
| NCI-H358 | G12C | Lung cancer | RPMI-1640 medium supplemented with 10% fetal bovine serum |
| MIA PaCa-2 | G12C | Pancreatic cancer | Dulbecco's modified Eagle medium (Sigma-Aldrich) supplemented with 10% fetal bovine serum and 2.5% horse serum (gibco) |

(continued)

| Cell name | KRAS mutation | Cancer type | Medium |
|---|---|---|---|
| NCI-H441 | G12V | Lung cancer | RPMI-1640 medium supplemented with 10% fetal bovine serum |
| NCI-H1373 | G12C | Lung cancer | RPMI-1640 medium supplemented with 10% fetal bovine serum, 2.5 g/L D-(+)-glucose solution, 10 mmol/L HEPES (all, Sigma-Aldrich), and 1 mmol/L sodium pyruvate (gibco) |
| HCC-1171 | G12C | Lung cancer | RPMI-1640 medium supplemented with 10% fetal bovine serum and 25 mmol/L HEPES (Sigma-Aldrich) |
| NCI-H23 | G12C | Lung cancer | RPMI-1640 medium supplemented with 10% fetal bovine serum, 2.5 g/L D-(+)-glucose solution, 10 mmol/L HEPES (all, Sigma-Aldrich), and 1 mmol/L sodium pyruvate (gibco) |
| A-427 | G12D | Lung cancer | Eagle's minimum essential medium (Nacalai Tesque) supplemented with 10% fetal bovine serum |
| NCI-H1944 | G13D | Lung cancer | RPMI-1640 medium supplemented with 10% fetal bovine serum, 2.5 g/L D-(+)-glucose solution, 10 mmol/L HEPES (all, Sigma-Aldrich), and 1 mmol/L sodium pyruvate (gibco) |
| GP2d | G12D | Colorectal cancer | Dulbecco's modified Eagle medium (Nacalai Tesque) supplemented with 10% fetal bovine serum |
| HPAC | G12D | Pancreatic cancer | Dulbecco's modified Eagle medium/Ham's F-12 medium (Nacalai Tesque) supplemented with 5% fetal bovine serum, 2 μg/mL human insulin (Thermo Fisher Scientific), 5 μg/mL human transferrin (Sigma-Aldrich), 40 ng/mL hydrocortisone (Sigma-Aldrich), and 10 ng/mL human epithelial growth factor (R&D Systems) |
| RPMI-1640 medium: Sigma-Aldrich or Nacalai Tesque<br>Fetal bovine serum: Corning or Sigma-Aldrich | | | |

$$\text{Cell proliferation inhibitory activity (\%)} = (1 - (T - V_0) / (V - V_0)) \times 100 \ldots (1)$$

T: ATP signal value after a lapse of the predetermined period of the compound treatment well

V: ATP signal value after a lapse of the predetermined period of the dimethyl sulfoxide treatment well

$V_0$: ATP signal value of a cell-uninoculated well

The cell proliferation inhibitory activity was plotted against the concentrations of the combined-use compound (here, sotorasib or adagrasib) or the compound 1 added (not shown) to calculate a concentration at which the combined-use compound or the compound 1 exhibited 80% cell proliferation inhibitory activity ($IC_{80}$).

In order to evaluate the effects of combined treatment, an isobologram was used. The results are shown in Figures 4(a) to 4(f). Figures 4(a) and 4(b) are isobolograms showing results of evaluating cell proliferation inhibitory activity using the cell line NCI-H2122. Figures 4(c) and 4(d) are isobolograms showing results of evaluating cell proliferation inhibitory activity using the cell line NCI-H358. Figures 4(e) and 4(f) are isobolograms showing results of evaluating cell proliferation inhibitory activity using the cell line MIA PaCa-2. In Figures 4(a), 4(c), and 4(e), sotorasib was used as the combined-use compound in the treatment. In Figures 4(b), 4(d), and 4(f), adagrasib was used as the combined-use compound in the treatment. In Figures 4(a) to 4(f), the abscissa of each graph depicts the final concentration (μmol/L) of the compound 1, and the ordinate thereof depicts the final concentration (μmol/L) of sotorasib or adagrasib. The broken lines in Figures 4(a) to 4(f) depict all combinations of the concentrations of the compound 1 and the concentrations of sotorasib or adagrasib in which the interaction between the compound 1 and sotorasib or adagrasib was additive. $IC_{80}$ of sotorasib or adagrasib was plotted against the concentrations of the compound 1 in the graphs. When these plots were positioned below the broken lines of the isobolograms, the combination of the compound 1 and sotorasib or adagrasib was confirmed to have synergistic effects.

A concentration at which the combined-use compound (here, TNO155, RMC-4550, or GDC-1971) or the compound 1 exhibited 50% cell proliferation inhibitory activity ($IC_{50}$) was calculated. $IC_{50}$ was also analyzed with isobolograms in the same manner as in $IC_{80}$. The analysis results using isobolograms are shown in Figures 5(a) to 16(a). Each drawing of Figure 5 shows results of treating the cell line NCI-H2122 using TNO155 as the combined-use compound. Each drawing of Figure 6 shows results of treating the cell line NCI-H358 using TNO155 as the combined-use compound. Each drawing of Figure 7 shows results of treating the cell line NCI-H2122 using RMC-4550 as the combined-use compound. Each drawing of Figure 8 shows results of treating the cell line NCI-H358 using RMC-4550 as the combined-use compound. Each drawing of Figure 9 shows results of treating the cell line NCI-H1373 using TNO155 as the combined-use compound. Each drawing of Figure 10 shows results of treating the cell line HCC-1171 using TNO155 as the combined-use compound. Each drawing of Figure 11 shows results of treating the cell line NCI-H23 using TNO155 as the combined-use compound. Each drawing of Figure 12 shows results of treating the cell line NCI-H1373 using GDC-1971 as the combined-use compound. Each drawing of Figure 13 shows results of treating the cell line A-427 using GDC-1971 as the combined-use compound. Each drawing of Figure 14 shows results of treating the cell line NCI-H1944 using GDC-1971 as the combined-use compound. Each drawing of Figure 15 shows results of treating the cell line GP2d using GDC-1971 as the combined-use compound. Each drawing of Figure 16 shows results of treating the cell line HPAC using GDC-1971 as the combined-use compound. Figures 5(a) to 16(a) revealed that use in combination of the compound 1 and the combined-use compound exhibits synergistic proliferation inhibitory effects because the plots (solid lines and/or dots) were positioned below the broken lines.

Use in combination of the compound 1 and the combined-use compound (here, TNO155, RMC-4550, or GDC-1971) was evaluated for its effects using a Bliss independence model. First, a Bliss index ($E_{Bliss}$) was calculated according to the expression (2) given below. $E_A$ represents cell proliferation inhibitory activity (%) when the compound 1 was used at certain concentration C in single-agent treatment. $E_B$ represents cell proliferation inhibitory activity (%) when the combined-use compound was used at certain concentration C in single-agent treatment. $E_{Bliss}$ is a calculated value (theoretical value) at which use in combination of the compound 1 and the combined-use compound exhibits additive effects.

$$E_{Bliss} = E_A + E_B - (E_A \times E_B) / 100 \ldots (2)$$

Next, an excess over Bliss score (EOB) was determined according to the expression (3) given below. The excess over Bliss score (EOB) is the difference of cell proliferation inhibition $E_{A+B}$ (%) obtained by combined treatment with the compound 1 and the combined-use compound from the Bliss index obtained according to the expression (2). When EOB was a positive value, the combined treatment was confirmed to have synergistic effects.

$$EOB = E_{A+B} - E_{Bliss} \ldots (3)$$

EOB is shown in Figures 5(c) to 16(c). Many combinations of concentrations exhibited positive EOB as a whole, indicating that use in combination of the compound 1 and the combined-use compound has synergistic proliferation inhibitory effects.

(Example 3: *In vivo* antitumor activity evaluation)

The single-agent administration of the sodium salt of the compound 1, sotorasib, or adagrasib, and the combined administration of the sodium salt of the compound 1 and each of these molecular-targeted agents (combined-use compounds) were evaluated for their *in vivo* antitumor activity and relative body weight change. This evaluation employed xenograft mice in which a KRAS-G12C mutation-positive human cancer cell line MIA PaCa-2 or NCI-H2122 (both, ATCC) was transplanted. The xenograft mice were prepared by subcutaneously transplanting $5 \times 10^6$ human cells per mouse to nude mice. After cell transplantation, the mice were grouped (n = 8) when a tumor volume reached 200 to 340 $mm^3$.

The MIA PaCa-2-transplanted models received the single-agent administration or combined administration of the sodium salt of the compound 1 and sotorasib. The sodium salt of the compound 1 and/or sotorasib was orally administered once a day for 14 days. The sodium salt of the compound 1 was dissolved in 10% DMSO/10% Cremophor EL/15% HP-β-CD (2-hydroxypropyl-β-cyclodextrin)/15% polyethylene glycol-400/50% injectable distilled water (solvent A) for administration. Sotorasib was dissolved in 1% HPMC (hydroxypropyl methyl cellulose)/1% Tween 80/98% injectable distilled water (solvent B) for administration. The sodium salt of the compound 1 and the solvent B were administered at 0.25 mg/kg and 20 ml/kg, respectively, to the single-agent administration group of the sodium salt of the compound 1. Sotorasib and the solvent A were administered at 10 mg/kg and 20 ml/kg, respectively, to the single-agent administration group of sotorasib. The sodium salt of the compound 1 and sotorasib were administered at 0.25 mg/kg and 10 mg/kg, respectively, to the combined administration group of the sodium salt of the compound 1 and sotorasib. The

solvent A and the solvent B were administered at 20 ml/kg and 20 ml/kg, respectively, to a control group. The sodium salt of the compound 1, sotorasib, the solvent A, and/or the solvent B was orally administered once a day for 14 days. After the completion of the administration period, an observation period of 22 days was established.

The NCI-H2122-transplanted models received the single-agent administration or combined administration of the sodium salt of the compound 1 and adagrasib. The sodium salt of the compound 1 and/or adagrasib was orally administered once a day for 21 days. The sodium salt of the compound 1 was dissolved in the solvent A for administration. Adagrasib was dissolved in 50 mM citrate buffer (pH 5.0)/10%HP-β-CD (2-hydroxypropyl-β-cyclodextrin) prepared with injectable distilled water (solvent C) for administration. The sodium salt of the compound 1 and the solvent C were administered at 0.25 mg/kg or 0.75 mg/kg and 20 ml/kg, respectively, to the single-agent administration group of the sodium salt of the compound 1. Adagrasib and the solvent A were administered at 100 mg/kg and 10 ml/kg, respectively, to the single-agent administration group of adagrasib. The sodium salt of the compound 1 and adagrasib were administered at 0.25 mg/kg or 0.75 mg/kg and 100 mg/kg, respectively, to the combined administration group of the sodium salt of the compound 1 and adagrasib. The solvent A and the solvent C were administered at 10 ml/kg and 20 ml/kg, respectively, to a control group. The sodium salt of the compound 1, adagrasib, the solvent A, and/or the solvent C was orally administered once a day for 21 days.

The tumor volumes of the mice were calculated according to the expression (4) given below. The results of evaluating *in vivo* antitumor activity are shown in Figures 17(a) and 17(c). Figures 17(a) and 17(b) show the experimental results obtained using the cell line MIA PaCa-2, and Figures 17(c) and 17(d) show the experimental results obtained using the cell line NCI-H2122. The ordinates of Figures 17(a) and 17(c) depict the tumor volumes ($mm^3$), and the abscissas thereof depict the number of days after cell transplantation.

$$\text{Tumor volume } (mm^3) = 1/2 \times \text{Major axis (mm)} \times \text{Minor axis (mm)} \times \text{Minor axis (mm)} \ldots (4)$$

Figures 17(a) and 17(c) revealed that the single-agent administration group of the sodium salt of the compound 1, the single-agent administration group of sotorasib or adagrasib, and the combined administration group of the sodium salt of the compound 1 and sotorasib or adagrasib compared with the control group suppressed increase in tumor volume. The combined administration group compared with the single-agent administration group had a larger degree of suppression of increase in tumor volume, indicating that use in combination of the sodium salt of the compound 1 and sotorasib or adagrasib potentiates *in vivo* antitumor activity.

The body weights of the mice were measured. Figures 17(b) and 17(d) show relative body weight change in the mice based on the body weights on the start day of administration of the sodium salt of the compound 1, the combined-use compound, the solvent A, the solvent B and/or the solvent C. The ordinates of Figures 17(b) and 17(d) depict the relative body weight change (%), and the abscissas thereof depict the number of days after cell transplantation. In Figure 17(b), no marked decrease in body weight was found in the single-agent administration group and the combined administration group. In Figure 17(d), it was confirmed that decrease in body weight was alleviated in the combined administration group compared with the single-agent administration group.

(Example 4: *In vivo* antitumor activity evaluation)

Use in combination of the sodium salt of the compound 1 and bevacizumab was evaluated for its *in vivo* antitumor activity using nude mice in which a KRAS mutation-positive human cancer cell line NCI-H441 (ATCC) was transplanted and SCID mice in which a KRAS mutation-positive human cancer cell line OV56 (ECACC) was transplanted. The cells of each line were subcutaneously transplanted at $5 \times 10^6$ per mouse to the mice. The mice were grouped (n = 5) when a tumor volume reached 200 to 300 $mm^3$.

The NCI-H441-transplanted models and the OV56-transplanted models received the single-agent administration or combined administration of the sodium salt of the compound 1 and bevacizumab. The sodium salt of the compound 1 was dissolved in the solvent A for administration. Bevacizumab was diluted with physiological saline for administration.

Administration to the NCI-H441-transplanted models was performed as described below. The sodium salt of the compound 1 was administered at 0.0625 mg/kg to the single-agent administration group of the sodium salt of the compound 1. Bevacizumab was administered at 2.5 mg/kg to the single-agent administration group of bevacizumab. The sodium salt of the compound 1 and bevacizumab were administered at 0.0625 mg/kg and 2.5 mg/kg, respectively, to the combined administration group of the sodium salt of the compound 1 and bevacizumab. The solvent A was administered at 20 ml/kg to a control group. The sodium salt of the compound 1 and the solvent A were orally administered once a day for 10 days. Bevacizumab was administered by intraperitoneal injection at a frequency of twice a week.

Administration to the OV56-transplanted models was performed as described below. The sodium salt of the compound 1 and physiological saline were administered at 0.25 mg/kg and 10 ml/kg, respectively, to the single-agent administration group of the sodium salt of the compound 1. Bevacizumab and the solvent A were administered at 2.5 mg/kg

and 20 ml/kg, respectively, to the single-agent administration group of bevacizumab. The sodium salt of the compound 1 and bevacizumab were administered at 0.25 mg/kg and 2.5 mg/kg, respectively, to the combined administration group of the sodium salt of the compound 1 and bevacizumab. The solvent A and physiological saline were administered at 20 ml/kg and 10 ml/kg, respectively, to a control group. The sodium salt of the compound 1 and the solvent A were orally administered once a day for 10 days. Bevacizumab and physiological saline were administered by intraperitoneal injection at a frequency of twice a week.

The tumor volumes of the mice were calculated according to the expression (4). The results of evaluating *in vivo* antitumor activity are shown in Figures 18(a) and 18(b). Figure 18(a) shows the experimental results obtained using the cell line NCI-H441, and Figure 18(b) shows the experimental results obtained using the cell line OV56. The ordinates of Figures 18(a) and 18(b) depict the tumor volumes ($mm^3$), and the abscissas thereof depict the number of days after cell transplantation.

Figures 18(a) and 18(b) revealed that the single-agent administration group of the sodium salt of the compound 1, the single-agent administration group of bevacizumab, and the combined administration group of the sodium salt of the compound 1 and bevacizumab compared with the control group suppressed increase in tumor volume. The combined administration group compared with the single-agent administration group had a larger degree of suppression of increase in tumor volume, indicating that use in combination of the sodium salt of the compound 1 and bevacizumab potentiates *in vivo* antitumor activity.

(Example 5: *In vivo* antitumor activity evaluation)

Use in combination of the sodium salt of the compound 1 and TNO155 was evaluated for its *in vivo* antitumor activity using nude mice in which any of KRAS mutation-positive human cancer cell lines NCI-H1373, NCI-H358 and NCI-H441 (all, ATCC) was transplanted. The NCI-H1373 cells, the NCI-H358 cells, and the NCI-H441 cells were subcutaneously transplanted at $5 \times 10^6$, $1 \times 10^7$, and $5 \times 10^6$, respectively, per mouse to the mice. The mice were grouped (n = 5) when a tumor volume reached approximately 150 to 300 $mm^3$.

The NCI-H1373-, NCI-H358- or NCI-H441-transplanted models received the single-agent administration or combined administration of the sodium salt of the compound 1 and TNO155. The sodium salt of the compound 1 was dissolved in solvent A for administration. TNO155 was diluted with 0.5% Tween 80 and 0.5% methyl cellulose prepared with injectable distilled water (solvent D) for administration.

Administration to the NCI-H1373-transplanted models was performed as described below. The sodium salt of the compound 1 and the solvent D were administered at 0.25 mg/kg and 10 ml/kg, respectively, to the single-agent administration group of the sodium salt of the compound 1. TNO155 and 10% DMSO and 5% Cremophor EL prepared with injectable distilled water (solvent E) were administered at 10 mg/kg and 10 ml/kg, respectively, to the single-agent administration group of TNO155. The sodium salt of the compound 1 and TNO155 were administered at 0.25 mg/kg and 10 mg/kg, respectively, to the combined administration group of the sodium salt of the compound 1 and TNO155. The solvent E and the solvent D were administered at 10 ml/kg and 10 ml/kg, respectively, to a control group. The sodium salt of the compound 1 and the solvent E were orally administered once a day for 30 days. TNO155 and the solvent D were orally administered twice a day for 30 days.

Administration to the NCI-H358-transplanted models was performed as described below. The sodium salt of the compound 1 and the solvent D were administered at 0.25 mg/kg and 10 ml/kg, respectively, to the single-agent administration group of the sodium salt of the compound 1. TNO155 and the solvent A were administered at 5 mg/kg and 10 ml/kg, respectively, to the single-agent administration group of TNO155. The sodium salt of the compound 1 and TNO155 were administered at 0.25 mg/kg and 5 mg/kg, respectively, to the combined administration group of the sodium salt of the compound 1 and TNO155. The solvent A and the solvent D were administered at 10 ml/kg and 10 ml/kg, respectively, to a control group. The sodium salt of the compound 1 and the solvent A were orally administered once a day for 21 days. TNO155 and the solvent D were orally administered twice a day for 21 days.

Administration to the NCI-H441-transplanted models was performed as described below. The sodium salt of the compound 1 and the solvent D were administered at 0.125 mg/kg or 0.25 mg/kg and 10 ml/kg, respectively, to the single-agent administration group of the sodium salt of the compound 1. TNO155 and the solvent A were administered at 5 mg/kg or 10 mg/kg and 10 ml/kg, respectively, to the single-agent administration group of TNO155. The sodium salt of the compound 1 and TNO155 were administered at 0.125 mg/kg or 0.25 mg/kg and 5 mg/kg or 10 mg/kg, respectively, to the combined administration group of the sodium salt of the compound 1 and TNO155. The solvent A and the solvent D were administered at 10 ml/kg and 10 ml/kg, respectively, to a control group. The sodium salt of the compound 1 and the solvent A were orally administered once a day for 13 days. TNO155 and the solvent D were orally administered twice a day for 13 days.

The tumor volumes of the mice were calculated according to the expression (4). The results of evaluating *in vivo* antitumor activity are shown in Figures 19A, 19B, and 19C. Figure 19A shows the experimental results obtained using the cell line NCI-H1373, Figure 19B shows the experimental results obtained using the cell line NCI-H358, and 19C show the

experimental results obtained using the cell line NCI-H441. Figure 19C(a) shows the experimental results obtained using the sodium salt of the compound 1 at a dose of 0.125 mg/kg and TNO155 at a dose of 5 mg/kg, Figure 19C(b) shows the experimental results obtained using the sodium salt of the compound 1 at a dose of 0.125 mg/kg and TNO155 at a dose of 10 mg/kg, Figure 19C(c) shows the experimental results obtained using the sodium salt of the compound 1 at a dose of 0.25 mg/kg and TNO155 at a dose of 5 mg/kg, and Figure 19C(d) shows the experimental results obtained using the sodium salt of the compound 1 at a dose of 0.25 mg/kg and TNO155 at a dose of 10 mg/kg. In Figures 19C(a) to 19C(d), "Compound 1" depicts the single-agent administration group of the sodium salt of the compound 1, "TNO" depicts the single-agent administration group of TNO155, "Combo" depicts the combined administration group of the sodium salt of the compound 1 and TNO155, and "Control" depicts the control group. The ordinates of Figures 19A, 19B, and 19Cdepict the tumor volumes ($mm^3$), and the abscissas thereof depict the number of days after cell transplantation or after the start of administration.

Figures 19A, 19B, and 19C revealed that the single-agent administration group of the sodium salt of the compound 1, the single-agent administration group of TNO155, and the combined administration group of the sodium salt of the compound 1 and TNO155 compared with the control group suppressed increase in tumor volume. The combined administration group compared with the single-agent administration group had a larger degree of suppression of increase in tumor volume, indicating that use in combination of the sodium salt of the compound 1 and TNO155 potentiates *in vivo* antitumor activity.

(Example 6: *In vivo* antitumor activity evaluation)

Use in combination of the sodium salt of the compound 1 and an anti-PD-L1 antibody was evaluated for its *in vivo* antitumor activity using BALB/C mice in which a KRAS mutation-positive mouse colon cancer cell line CT26 (Crown Bioscience) was transplanted and C57BL/6J mice in which a KRAS mutation-positive mouse lung cancer tissue slice mLU6054 (Crown Bioscience) was transplanted. The CT26 cells and the mLU6054 tissue slice were subcutaneously transplanted at $5 \times 10^5$ and a diameter of 2 to 3 $mm^3$, respectively, per mouse to the mice. The mice were grouped (n = 8) when a tumor volume reached approximately 60 to 85 $mm^3$.

The CT26- or mLU6054-transplanted models received the single-agent administration or combined administration of the sodium salt of the compound 1 and the anti-PD-L1 antibody. The sodium salt of the compound 1 was dissolved in solvent E for administration. The anti-PD-L1 antibody was diluted with PBS for administration.

Administration to the CT26- or mLU6054-transplanted models was performed as described below. The sodium salt of the compound 1 and PBS were administered at 1 mg/kg and 10 ml/kg, respectively, to the single-agent administration group of the sodium salt of the compound 1. The anti-PD-L1 antibody and the solvent E were administered at 10 mg/kg and 20 ml/kg, respectively, to the single-agent administration group of the anti-PD-L1 antibody. The sodium salt of the compound 1 and the anti-PD-L1 antibody were administered at 1 mg/kg and 10 mg/kg, respectively, to the combined administration group of the sodium salt of the compound 1 and the anti-PD-L1 antibody. The solvent E and PBS were administered at 20 ml/kg and 10 ml/kg, respectively, to a control group. The sodium salt of the compound 1 and the solvent E were orally administered once a day for 21 days. The anti-PD-L1 antibody and PBS were administered by intravenous injection twice a week for 21 days.

The tumor volumes of the mice were calculated according to the expression (4). The results of evaluating *in vivo* antitumor activity are shown in Figures 20(a) and 20(b). Figure 20(a) shows the experimental results obtained using CT26, and Figure 20(b) show the experimental results obtained using mLU6054. The ordinates of Figures 20(a) and 20(b) depict the tumor volumes ($mm^3$), and the abscissas thereof depict the number of days after the start of administration.

Figures 20(a) and 20(b) revealed that the single-agent administration group of the sodium salt of the compound 1, the single-agent administration group of the anti-PD-L1 antibody, and the combined administration group of the sodium salt of the compound 1 and the anti-PD-L1 antibody compared with the control group suppressed increase in tumor volume. The combined administration group compared with the single-agent administration group had a larger degree of suppression of increase in tumor volume, indicating that use in combination of the sodium salt of the compound 1 and the anti-PD-L1 antibody potentiates *in vivo* antitumor activity.

## Claims

1. A drug for treating or preventing cancer, the drug comprising a compound represented by the following formula (1), a salt thereof or a solvate thereof and being used in combination with a molecular-targeted agent, wherein the molecular-targeted agent is at least one member selected from the group consisting of a KRAS-G12C selective inhibitor, a SHP2 inhibitor, a VEGF inhibitor and a PD-1 axis binding antagonist.

[Formula 1]

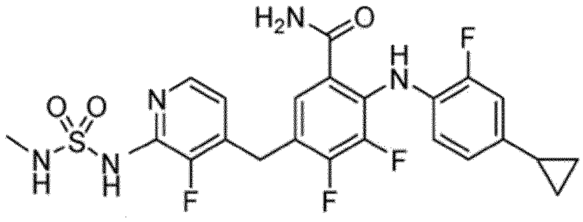

(1)

2. A drug for treating or preventing cancer, the drug comprising a molecular-targeted agent and being used in combination with a compound represented by the following formula (1), a salt thereof or a solvate thereof, wherein the molecular-targeted agent is at least one member selected from the group consisting of a KRAS-G12C selective inhibitor, a SHP2 inhibitor, a VEGF inhibitor and a PD-1 axis binding antagonist.

[Formula 2]

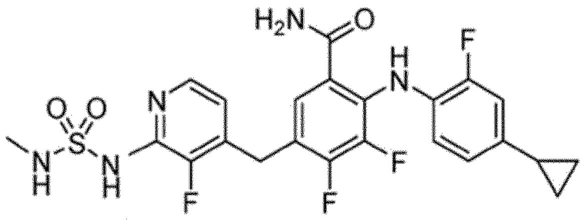

(1)

3. The drug according to claim 1 or 2, wherein the compound represented by the formula (1), the salt thereof or the solvate thereof and the molecular-targeted agent are provided as a kit.

4. The drug according to any one of claims 1 to 3, wherein the compound represented by the formula (1), the salt thereof or the solvate thereof and the molecular-targeted agent are used in combination concurrently or separately.

5. The drug according to any one of claims 1 to 4, wherein the compound represented by the formula (1), the salt thereof or the solvate thereof and the molecular-targeted agent are administered as a combination drug.

6. The drug according to any one of claims 1 to 5, wherein the molecular-targeted agent is a KRAS-G12C selective inhibitor, a SHP2 inhibitor, a VEGF inhibitor, or a PD-1 axis binding antagonist.

7. The drug according to any one of claims 1 to 6, wherein the molecular-targeted agent is a KRAS-G12C selective inhibitor.

8. The drug according to any one of claims 1 to 6, wherein the molecular-targeted agent is a SHP2 inhibitor.

9. The drug according to any one of claims 1 to 6, wherein the molecular-targeted agent is a VEGF inhibitor.

10. The drug according to any one of claims 1 to 6, wherein the molecular-targeted agent is a PD-1 axis binding antagonist.

11. The drug according to any one of claims 1 to 7, wherein the KRAS-G12C selective inhibitor is at least one member selected from the group consisting of sotorasib, adagrasib, a salt thereof, and a solvate thereof.

12. The drug according to any one of claims 1 to 6 and 8, wherein the SHP2 inhibitor is at least one member selected from the group consisting of RMC-4550, TNO155, SHP099, NSC-87877, RMC-4630, GDC-1971, a salt thereof, and a solvate thereof.

13. The drug according to any one of claims 1 to 6 and 9, wherein the VEGF inhibitor is bevacizumab.

14. The drug according to any one of claims 1 to 6 and 10, wherein the PD-1 axis binding antagonist is an anti-PD-L1 antibody.

**15.** The drug according to any one of claims 1 to 14, wherein the compound represented by the formula (1), the salt thereof or the solvate thereof is the salt of the compound represented by the formula (1).

**16.** The drug according to claim 15, wherein the salt is a sodium salt.

**17.** The drug according to any one of claims 1 to 14, wherein the compound represented by the formula (1), the salt thereof or the solvate thereof is the compound represented by the formula (1).

**18.** The drug according to any one of claims 1 to 17, wherein the cancer is at least one member selected from the group consisting of lung cancer, esophageal cancer, stomach cancer, colorectal cancer, uterine cancer, ovarian cancer, pancreatic cancer, bladder cancer, thyroid gland cancer, skin cancer, head and neck cancer, leukemia, malignant lymphoma and multiple myeloma.

*Fig.1*

NCI-H2122
(KRAS G12C mt, NSCLC)

Compound 1 : 300 nM
Sotorasib: 200 nM
Adagrasib: 1,000 nM

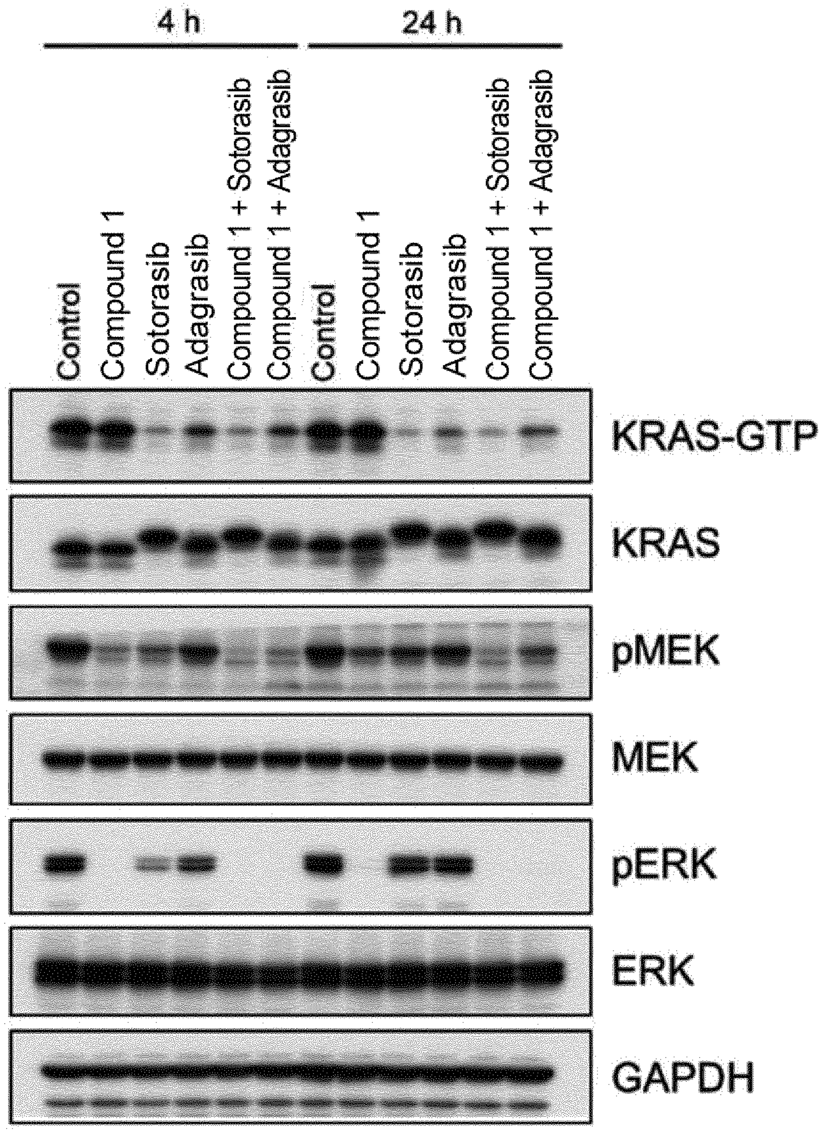

# Fig.2

NCI-H1373 (KRAS G12C, NSCLC)
Compound 1: 30 nM, TNO155: 1,000 nM

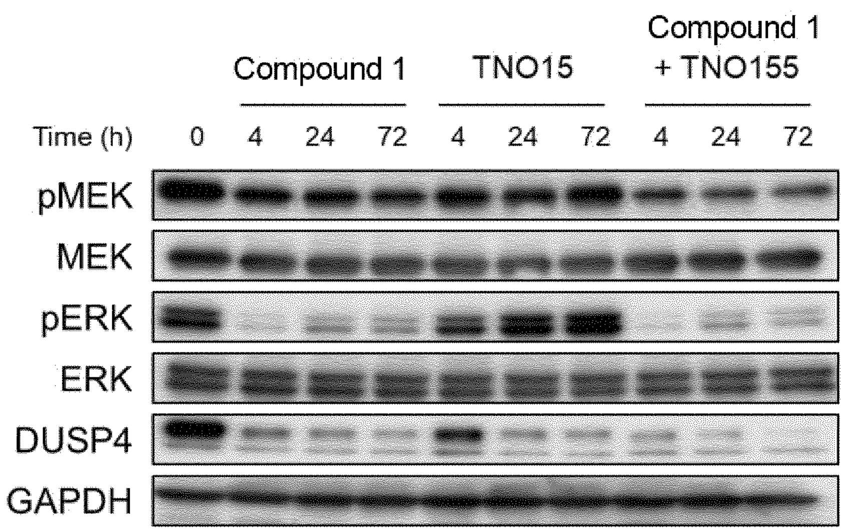


NCI-H441 (KRAS G12V, NSCLC)
Compound 1: 10 nM, TNO155: 1,000 nM

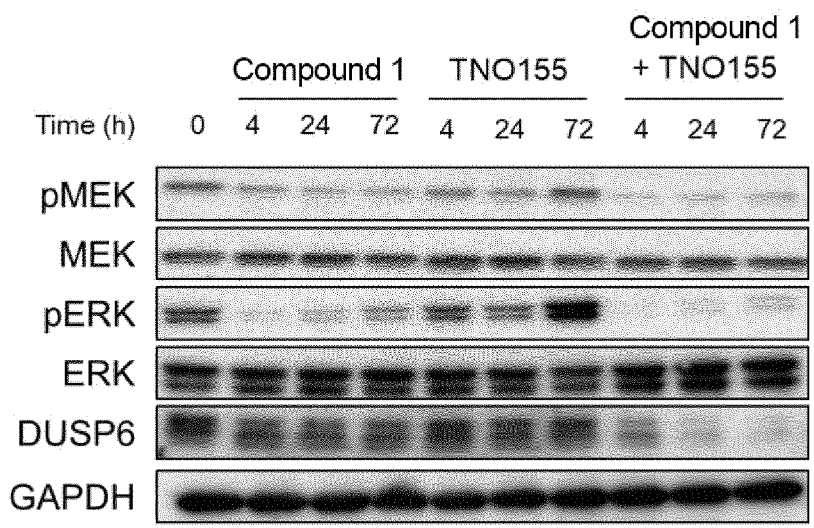

Fig.3

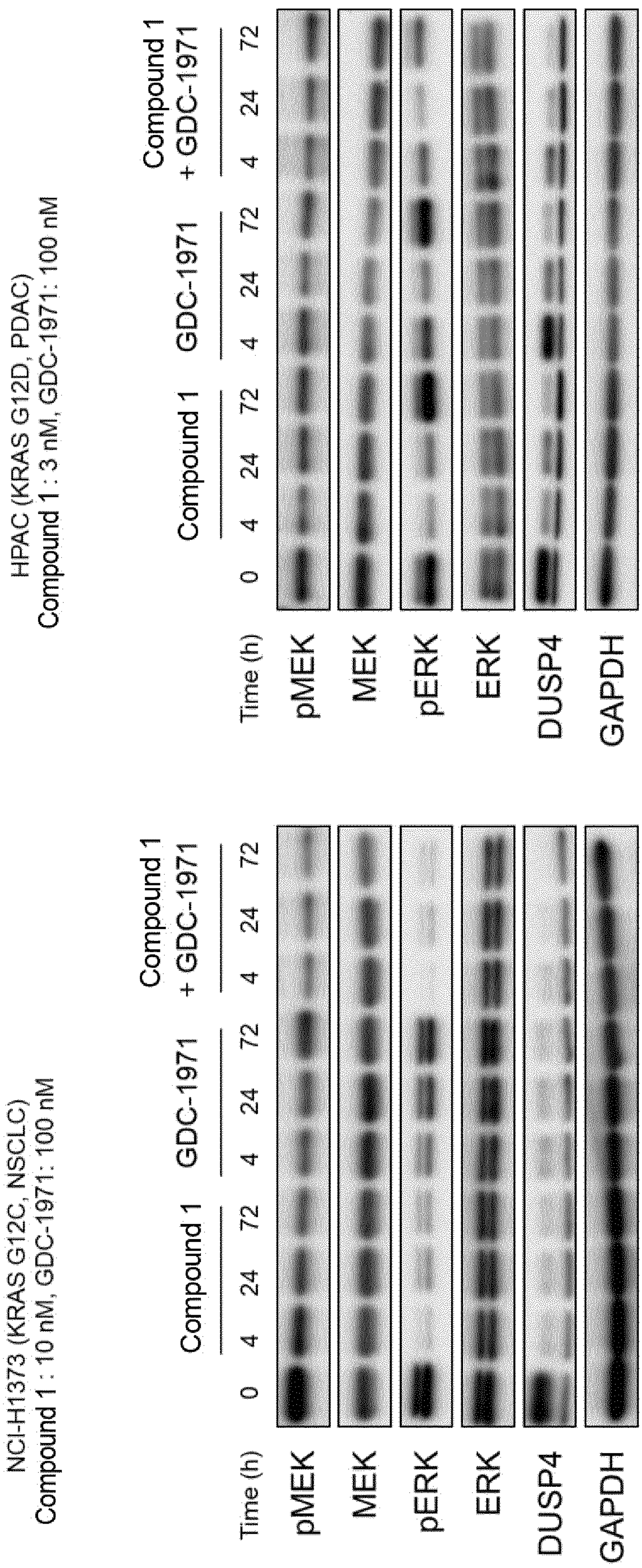
NCI-H1373 (KRAS G12C, NSCLC)
Compound 1 : 10 nM, GDC-1971: 100 nM
Compound 1
GDC-1971
Compound 1
+ GDC-1971
Time (h)
0
4
24
72
4
24
72
4
24
72
pMEK
MEK
pERK
ERK
DUSP4
GAPDH
HPAC (KRAS G12D, PDAC)
Compound 1 : 3 nM, GDC-1971: 100 nM
Compound 1
GDC-1971
Compound 1
+ GDC-1971
Time (h)
0
4
24
72
4
24
72
4
24
72
pMEK
MEK
pERK
ERK
DUSP4
GAPDH

Fig.4

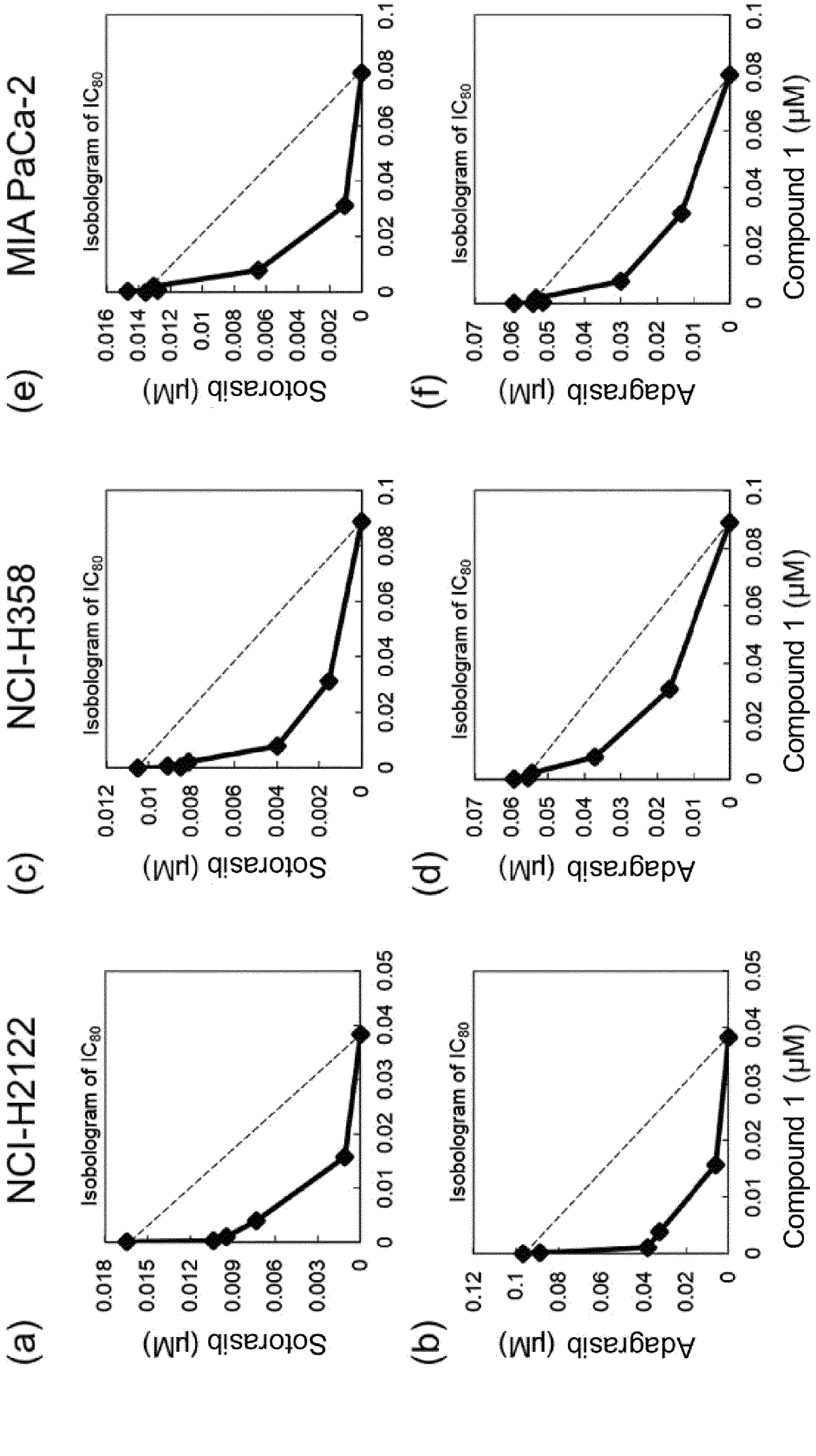
(a)
NCI-H2122
Isobologram of $IC_{80}$
Sotorasib (μM)
0.018
0.015
0.012
0.009
0.006
0.003
0
0.01
0.02
0.03
0.04
0.05
(b)
Isobologram of $IC_{80}$
Adagrasib (μM)
0.12
0.1
0.08
0.06
0.04
0.02
Compound 1 (μM)
(c)
NCI-H358
Isobologram of $IC_{80}$
Sotorasib (μM)
0.012
0.01
0.008
0.006
0.004
0.002
0.1
(d)
Isobologram of $IC_{80}$
Adagrasib (μM)
0.07
0.06
0.05
Compound 1 (μM)
(e)
MIA PaCa-2
Isobologram of $IC_{80}$
Sotorasib (μM)
0.016
0.014
(f)
Isobologram of $IC_{80}$
Adagrasib (μM)
Compound 1 (μM)

# Fig.5

(a) Isobologram

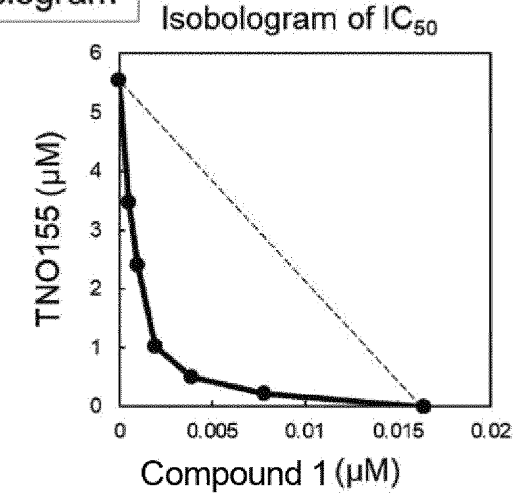


(b) Proliferation inhibition (%)

| TNO155 (μM) \ Compound 1 (μM) | 0.0000 | 0.0005 | 0.0010 | 0.0020 | 0.0039 | 0.0078 | 0.0156 | 0.0313 | 0.0625 | 0.1250 |
|---|---|---|---|---|---|---|---|---|---|---|
| 0.0000 | 0.0 | 0.3 | 3.5 | 6.7 | 13.9 | 23.5 | 47.8 | 68.8 | 92.9 | 97.4 |
| 0.0391 | -0.7 | 3.8 | 8.5 | 9.1 | 13.4 | 30.0 | 49.7 | 72.1 | 95.2 | 97.7 |
| 0.1563 | 5.0 | 7.2 | 12.1 | 16.6 | 25.1 | 38.4 | 64.0 | 86.3 | 96.6 | 98.2 |
| 0.6250 | 17.7 | 23.9 | 25.4 | 38.3 | 53.6 | 63.2 | 87.6 | 95.9 | 97.9 | 98.2 |
| 2.5000 | 34.1 | 47.2 | 52.1 | 70.5 | 82.9 | 89.8 | 98.0 | 99.3 | 99.9 | 98.6 |
| 10.0000 | 61.3 | 65.3 | 73.2 | 84.5 | 91.0 | 93.6 | 96.0 | 98.2 | 99.4 | 98.7 |

(c) Excess over Bliss score

| TNO155 (μM) \ Compound 1 (μM) | 0.0000 | 0.0005 | 0.0010 | 0.0020 | 0.0039 | 0.0078 | 0.0156 | 0.0313 | 0.0625 | 0.1250 |
|---|---|---|---|---|---|---|---|---|---|---|
| 0.0000 | 0.0 | 0.0 | 0.0 | 0.0 | 0.0 | 0.0 | 0.0 | 0.0 | 0.0 | 0.0 |
| 0.0391 | 0.0 | 4.2 | 5.7 | 3.1 | 0.1 | 7.0 | 2.3 | 3.5 | 2.3 | 0.3 |
| 0.1563 | 0.0 | 2.0 | 3.8 | 5.2 | 6.9 | 11.1 | 13.6 | 16.0 | 3.4 | 0.6 |
| 0.6250 | 0.0 | 6.0 | 4.8 | 15.1 | 24.4 | 26.2 | 30.5 | 21.5 | 3.8 | 0.3 |
| 2.5000 | 0.0 | 13.0 | 15.7 | 32.0 | 39.6 | 40.3 | 32.4 | 19.8 | 4.5 | 0.3 |
| 10.0000 | 0.0 | 3.9 | 10.5 | 20.6 | 24.3 | 23.2 | 16.2 | 10.3 | 2.1 | -0.3 |

# Fig.6

(a) Isobologram

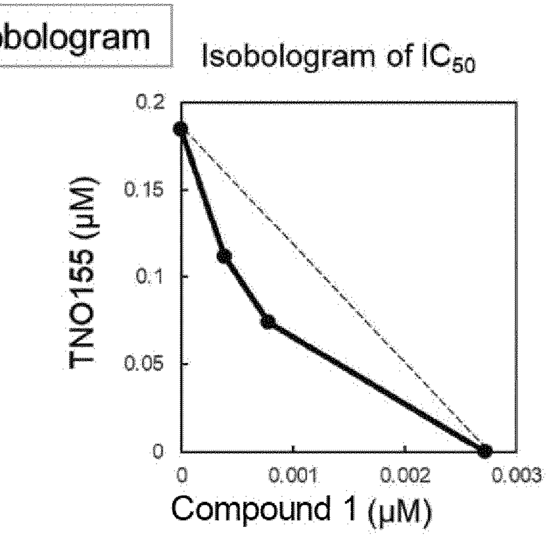


(b) Proliferation inhibition (%)

| TNO155 (μM) \ Compound 1 (μM) | 0.0000 | 0.0004 | 0.0008 | 0.0016 | 0.0031 | 0.0063 | 0.0125 | 0.0250 | 0.0500 | 0.1000 |
|---|---|---|---|---|---|---|---|---|---|---|
| 0.0000 | 0.0 | 11.7 | 24.0 | 38.2 | 55.7 | 70.5 | 80.2 | 84.2 | 88.5 | 91.0 |
| 0.0391 | 13.2 | 29.2 | 36.3 | 54.4 | 63.6 | 80.2 | 85.6 | 88.2 | 91.9 | 94.0 |
| 0.1563 | 47.4 | 57.1 | 65.1 | 72.6 | 76.8 | 88.5 | 91.1 | 93.5 | 95.1 | 95.7 |
| 0.6250 | 81.3 | 81.3 | 87.2 | 89.3 | 90.9 | 93.8 | 96.9 | 96.6 | 98.0 | 97.6 |
| 2.5000 | 88.6 | 92.6 | 93.4 | 94.4 | 95.4 | 97.2 | 97.9 | 99.0 | 99.5 | 99.2 |
| 10.0000 | 86.1 | 89.3 | 90.9 | 93.5 | 95.5 | 97.2 | 98.0 | 98.8 | 98.9 | 98.9 |

(c) Excess over Bliss score

| TNO155 (μM) \ Compound 1 (μM) | 0.0000 | 0.0004 | 0.0008 | 0.0016 | 0.0031 | 0.0063 | 0.0125 | 0.0250 | 0.0500 | 0.1000 |
|---|---|---|---|---|---|---|---|---|---|---|
| 0.0000 | 0.0 | 0.0 | 0.0 | 0.0 | 0.0 | 0.0 | 0.0 | 0.0 | 0.0 | 0.0 |
| 0.0391 | 0.0 | 5.8 | 2.2 | 8.0 | 2.1 | 5.7 | 2.8 | 1.9 | 1.8 | 1.8 |
| 0.1563 | 0.0 | 3.6 | 5.1 | 5.1 | 0.2 | 4.0 | 1.6 | 1.8 | 1.2 | 0.4 |
| 0.6250 | 0.0 | -2.2 | 1.3 | 0.8 | -0.9 | -0.7 | 0.6 | -0.5 | 0.2 | -0.7 |
| 2.5000 | 0.0 | 2.7 | 2.1 | 1.5 | 0.4 | 0.6 | 0.2 | 0.8 | 0.8 | 0.2 |
| 10.0000 | 0.0 | 1.5 | 1.5 | 2.1 | 1.7 | 1.3 | 0.8 | 1.0 | 0.5 | 0.2 |

# Fig.7

(a) Isobologram

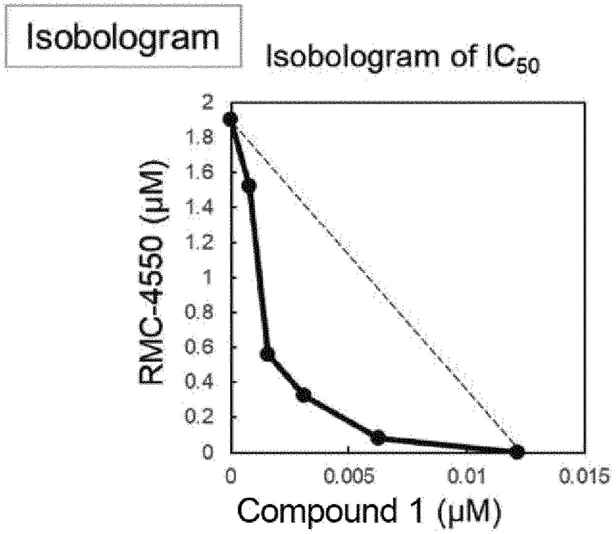


(b) Proliferation inhibition (%)

| RMC-4550 (µM) \ Compound 1 (µM) | 0.0000 | 0.0008 | 0.0016 | 0.0031 | 0.0063 | 0.0125 | 0.0250 | 0.0500 | 0.1000 | 0.2000 |
|---|---|---|---|---|---|---|---|---|---|---|
| 0.0000 | 0.0 | 5.1 | 6.9 | 15.5 | 31.7 | 47.3 | 68.6 | 91.7 | 98.7 | 97.8 |
| 0.0391 | 1.0 | 5.9 | 12.3 | 19.2 | 39.3 | 57.8 | 80.9 | 93.6 | 99.4 | 97.9 |
| 0.1563 | 15.4 | 16.1 | 25.9 | 39.2 | 59.4 | 82.0 | 83.8 | 98.4 | 99.8 | 98.1 |
| 0.6250 | 35.4 | 37.9 | 52.8 | 56.8 | 80.3 | 91.3 | 98.3 | 99.7 | 99.9 | 98.2 |
| 2.5000 | 46.9 | 59.6 | 78.5 | 84.1 | 91.9 | 97.1 | 99.5 | 99.9 | 99.9 | 98.6 |
| 10.0000 | 82.8 | 75.1 | 84.5 | 90.5 | 93.8 | 95.9 | 98.4 | 99.5 | 99.9 | 98.7 |

(c) Excess over Bliss score

| RMC-4550 (µM) \ Compound 1 (µM) | 0.0000 | 0.0008 | 0.0016 | 0.0031 | 0.0063 | 0.0125 | 0.0250 | 0.0500 | 0.1000 | 0.2000 |
|---|---|---|---|---|---|---|---|---|---|---|
| 0.0000 | 0.0 | 0.0 | 0.0 | 0.0 | 0.0 | 0.0 | 0.0 | 0.0 | 0.0 | 0.0 |
| 0.0391 | 0.0 | -0.2 | 4.5 | 2.8 | 7.0 | 10.0 | 12.0 | 1.8 | 0.6 | 0.2 |
| 0.1563 | 0.0 | -3.6 | 4.7 | 10.7 | 17.2 | 26.5 | 10.4 | 5.4 | 0.9 | 0.0 |
| 0.6250 | 0.0 | -0.8 | 13.0 | 11.4 | 24.5 | 25.4 | 18.5 | 5.0 | 0.7 | -0.4 |
| 2.5000 | 0.0 | 9.9 | 27.9 | 28.9 | 28.2 | 25.1 | 16.1 | 4.3 | 0.6 | -0.2 |
| 10.0000 | 0.0 | -8.5 | 0.5 | 5.0 | 5.6 | 5.0 | 3.7 | 0.9 | 0.1 | -0.9 |

# Fig.8

(a) Isobologram

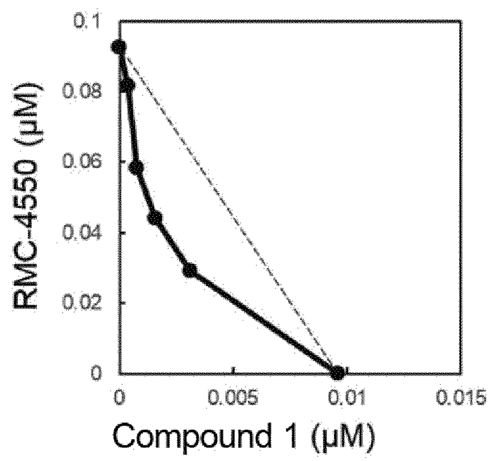


(b) Proliferation inhibition (%)

| RMC-4550 (μM) \ Compound 1 (μM) | 0.0000 | 0.0004 | 0.0008 | 0.0016 | 0.0031 | 0.0063 | 0.0125 | 0.0250 | 0.0500 | 0.1000 |
|---|---|---|---|---|---|---|---|---|---|---|
| 0.0000 | 0.0 | -3.1 | 10.3 | 11.8 | 22.2 | 42.9 | 55.3 | 75.0 | 84.3 | 86.7 |
| 0.0039 | -6.6 | -2.1 | 7.8 | 16.8 | 32.5 | 52.9 | 59.8 | 80.5 | 86.5 | 89.2 |
| 0.0156 | 13.7 | 17.1 | 22.1 | 34.2 | 37.5 | 52.8 | 70.1 | 76.1 | 84.4 | 91.2 |
| 0.0625 | 49.2 | 47.1 | 55.5 | 53.2 | 54.0 | 75.7 | 74.9 | 78.2 | 91.5 | 91.6 |
| 0.2500 | 73.2 | 77.0 | 79.7 | 80.3 | 82.4 | 86.6 | 92.6 | 95.0 | 95.1 | 95.9 |
| 1.0000 | 76.7 | 80.3 | 81.3 | 85.7 | 85.1 | 87.0 | 92.7 | 95.2 | 97.5 | 97.2 |

(c) Excess over Bliss score

| RMC-4550 (μM) \ Compound 1 (μM) | 0.0000 | 0.0004 | 0.0008 | 0.0016 | 0.0031 | 0.0063 | 0.0125 | 0.0250 | 0.0500 | 0.1000 |
|---|---|---|---|---|---|---|---|---|---|---|
| 0.0000 | 0.0 | 0.0 | 0.0 | 0.0 | 0.0 | 0.0 | 0.0 | 0.0 | 0.0 | 0.0 |
| 0.0039 | 0.0 | 7.8 | 3.4 | 10.8 | 15.4 | 13.7 | 7.4 | 7.1 | 3.3 | 3.3 |
| 0.0156 | 0.0 | 6.1 | -0.4 | 10.3 | 4.7 | 2.1 | 8.7 | -2.4 | -2.0 | 2.6 |
| 0.0625 | 0.0 | -0.5 | 1.1 | -2.0 | -6.4 | 4.8 | -2.4 | -9.1 | -0.5 | -1.7 |
| 0.2500 | 0.0 | 4.7 | 3.8 | 4.0 | 3.3 | 1.9 | 4.6 | 1.6 | -0.7 | -0.5 |
| 1.0000 | 0.0 | 4.3 | 2.2 | 6.2 | 3.2 | 0.3 | 3.2 | 1.0 | 1.1 | 0.3 |

# Fig.9

(a) Isobologram

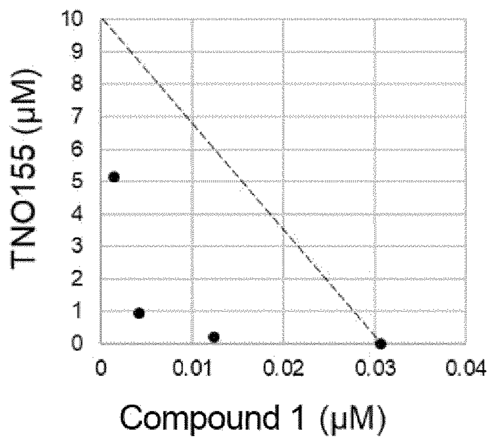


(b) Proliferation inhibition (%)

| TNO155 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | -5.5 | 0.7 | 3.2 | 14.5 | 34.9 | 57.1 | 66.5 | 82.8 | 91.4 |
| 0.0160 | -10.2 | -1.7 | 5.6 | 11.4 | 14.6 | 42.6 | 57.8 | 70.4 | 83.5 | 90.6 |
| 0.0800 | 12.8 | 10.6 | 16.9 | 17.4 | 25.7 | 43.4 | 60.2 | 73.1 | 89.5 | 92.5 |
| 0.4000 | 17.3 | 20.4 | 19.1 | 31.6 | 43.2 | 56.5 | 70.3 | 81.5 | 90.8 | 95.2 |
| 2.0000 | 28.0 | 30.6 | 27.7 | 40.9 | 55.0 | 74.8 | 78.5 | 90.0 | 93.8 | 96.1 |
| 10.0000 | 27.7 | 42.9 | 44.8 | 55.9 | 66.3 | 81.8 | 92.5 | 94.4 | 97.1 | 97.0 |

(c) Excess over Bliss score

| TNO155 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0160 | 0 | 15 | 15 | 18 | 9 | 14 | 5 | 7 | 2 | 0 |
| 0.0800 | 0 | 3 | 3 | 2 | 0 | 0 | -2 | 2 | 5 | 0 |
| 0.4000 | 0 | 8 | 1 | 12 | 14 | 10 | 6 | 9 | 5 | 2 |
| 2.0000 | 0 | 7 | -1 | 11 | 16 | 22 | 9 | 14 | 6 | 2 |
| 10.0000 | 0 | 19 | 17 | 26 | 28 | 29 | 24 | 19 | 10 | 3 |

# Fig.10

(a) Isobologram

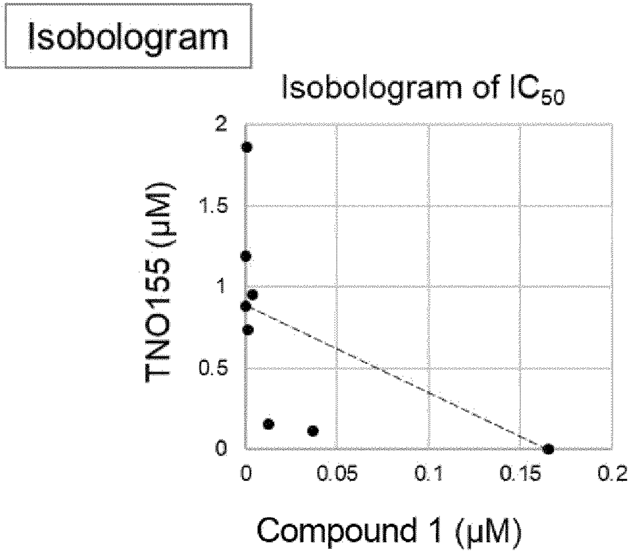


(b) Proliferation inhibition (%)

| TNO155 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | -26.7 | -10.5 | -1.4 | 15.5 | 27.2 | 24.1 | 48.2 | 63.6 | 62.7 |
| 0.0160 | 4.9 | 15.9 | 24.3 | 25.9 | 29.4 | 24.6 | 43.1 | 54.5 | 68.4 | 63.8 |
| 0.0800 | 25.4 | 16.3 | 0.7 | 26.5 | 32.6 | 43.1 | 45.0 | 65.3 | 72.2 | 52.1 |
| 0.4000 | 45.3 | 37.2 | 2.5 | 42.5 | 36.5 | 59.3 | 74.2 | 82.6 | 79.0 | 79.1 |
| 2.0000 | 53.8 | 55.7 | 55.6 | 64.5 | 67.7 | 82.1 | 86.3 | 85.7 | 93.8 | 90.5 |
| 10.0000 | 64.8 | 73.2 | 65.4 | 80.5 | 87.2 | 87.7 | 95.4 | 91.2 | 96.0 | 90.6 |

(c) Excess over Bliss score

| TNO155 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0160 | 0 | 36 | 29 | 22 | 10 | -6 | 15 | 4 | 3 | -1 |
| 0.0800 | 0 | 11 | -17 | 2 | -4 | -3 | 2 | 4 | -1 | -20 |
| 0.4000 | 0 | 6 | -37 | -2 | -17 | -1 | 16 | 11 | -1 | -1 |
| 2.0000 | 0 | 14 | 7 | 11 | 7 | 16 | 21 | 10 | 11 | 8 |
| 10.0000 | 0 | 18 | 4 | 16 | 17 | 13 | 22 | 9 | 9 | 4 |

# Fig.11

(a) Isobologram

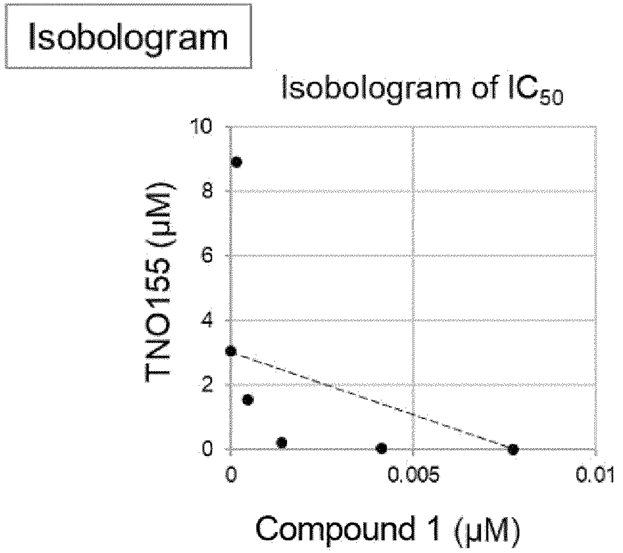


(b) Proliferation inhibition (%)

| TNO155 (µM) \ Compound 1 (µM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | -1.9 | 12.2 | 19.1 | 34.9 | 61.5 | 76.5 | 85.3 | 94.0 | 96.6 |
| 0.0160 | 1.6 | 3.9 | 11.8 | 15.7 | 38.7 | 66.8 | 76.9 | 86.3 | 94.6 | 96.7 |
| 0.0800 | 12.6 | 12.0 | 18.7 | 22.3 | 53.8 | 74.6 | 79.9 | 90.9 | 95.2 | 97.8 |
| 0.4000 | 31.2 | 32.3 | 36.3 | 54.3 | 70.9 | 77.9 | 87.2 | 92.8 | 95.5 | 97.4 |
| 2.0000 | 45.7 | 26.8 | 51.7 | 51.5 | 84.1 | 86.6 | 91.3 | 93.1 | 95.8 | 98.1 |
| 10.0000 | 57.9 | 54.3 | 65.4 | 58.8 | 85.7 | 88.1 | 92.4 | 93.8 | 96.3 | 96.9 |

(c) Excess over Bliss score

| TNO155 (µM) \ Compound 1 (µM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0160 | 0 | 4 | -2 | -5 | 3 | 5 | 0 | 1 | 0 | 0 |
| 0.0800 | 0 | 1 | -5 | -7 | 11 | 8 | 1 | 4 | 0 | 1 |
| 0.4000 | 0 | 2 | -3 | 10 | 16 | 4 | 3 | 3 | 0 | 0 |
| 2.0000 | 0 | -18 | -1 | -5 | 19 | 7 | 4 | 1 | -1 | 0 |
| 10.0000 | 0 | -3 | 2 | -7 | 13 | 4 | 2 | 0 | -1 | -2 |

# Fig.12

(a) Isobologram

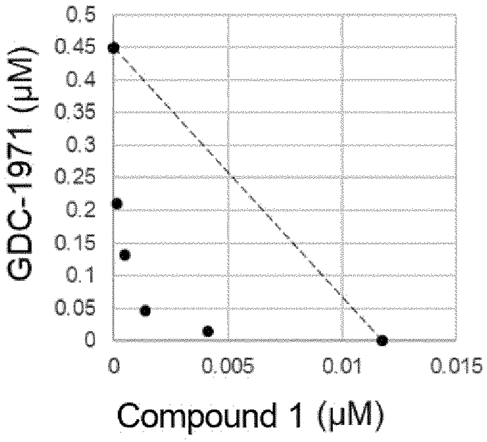


(b) Proliferation inhibition (%)

| GDC-1971 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | 0.2 | 2.6 | 11.6 | 31.4 | 52.7 | 68.6 | 80.9 | 89.8 | 93.8 |
| 0.0080 | 19.2 | 25.8 | 31.5 | 32.3 | 41.3 | 62.1 | 71.7 | 85.9 | 92.1 | 94.6 |
| 0.0400 | 35.0 | 40.8 | 43.1 | 50.0 | 59.8 | 74.7 | 82.9 | 90.4 | 94.0 | 96.1 |
| 0.2000 | 43.1 | 48.9 | 53.4 | 59.2 | 73.9 | 81.0 | 88.4 | 93.2 | 96.6 | 97.5 |
| 1.0000 | 54.7 | 56.8 | 58.1 | 68.2 | 75.1 | 86.7 | 92.1 | 95.7 | 98.4 | 98.0 |
| 5.0000 | 65.6 | 63.4 | 66.9 | 73.4 | 81.9 | 90.2 | 91.4 | 93.4 | 94.4 | 95.0 |

(c) Excess over Bliss score

| GDC-1971 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0080 | 0 | 7 | 10 | 4 | -3 | 0 | -3 | 1 | 0 | 0 |
| 0.0400 | 0 | 6 | 6 | 8 | 4 | 5 | 3 | 3 | 1 | 0 |
| 0.2000 | 0 | 6 | 9 | 10 | 13 | 8 | 6 | 4 | 2 | 1 |
| 1.0000 | 0 | 2 | 2 | 8 | 6 | 8 | 6 | 4 | 3 | 1 |
| 5.0000 | 0 | -2 | 0 | 4 | 6 | 6 | 2 | 0 | -2 | -3 |

# Fig.13

(a) Isobologram

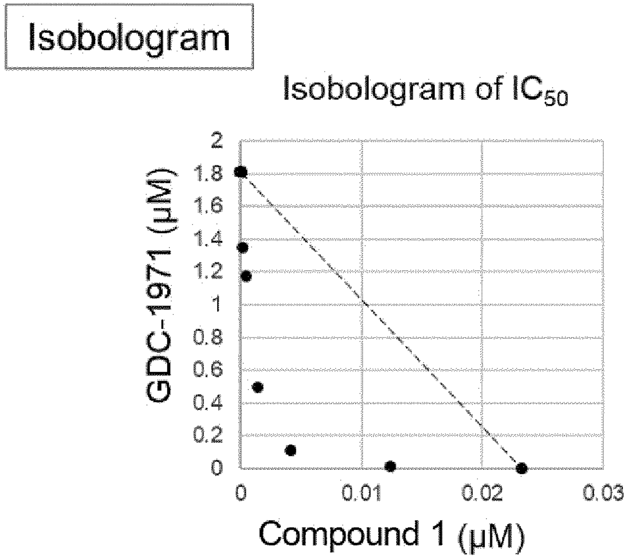


(b) Proliferation inhibition (%)

| GDC-1971 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | 3.1 | 8.0 | 14.2 | 24.9 | 42.0 | 49.7 | 81.7 | 88.4 | 93.6 |
| 0.0080 | 18.3 | 17.5 | 24.3 | 27.0 | 34.6 | 48.6 | 59.8 | 83.5 | 93.2 | 94.8 |
| 0.0400 | 25.9 | 27.6 | 30.0 | 36.9 | 43.1 | 55.3 | 69.0 | 89.0 | 94.2 | 95.7 |
| 0.2000 | 37.4 | 38.6 | 39.7 | 42.9 | 54.4 | 66.2 | 79.3 | 93.5 | 95.7 | 96.6 |
| 1.0000 | 41.0 | 45.4 | 47.0 | 55.0 | 63.4 | 74.4 | 86.7 | 95.5 | 97.1 | 97.2 |
| 5.0000 | 60.7 | 60.3 | 63.2 | 65.7 | 74.3 | 79.1 | 87.1 | 92.7 | 94.1 | 94.4 |

(c) Excess over Bliss score

| GDC-1971 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0080 | 0 | -3 | -1 | -3 | -4 | -4 | 1 | -2 | 3 | 0 |
| 0.0400 | 0 | -1 | -2 | 0 | -1 | -2 | 6 | 3 | 3 | 0 |
| 0.2000 | 0 | -1 | -3 | -3 | 1 | 3 | 11 | 5 | 3 | 1 |
| 1.0000 | 0 | 3 | 1 | 6 | 8 | 9 | 16 | 6 | 4 | 1 |
| 5.0000 | 0 | -2 | -1 | -1 | 4 | 2 | 7 | 0 | -1 | -3 |

# Fig.14

(a) Isobologram

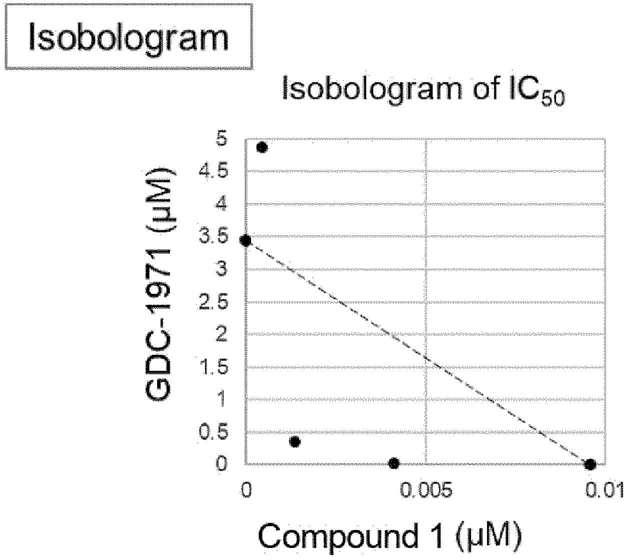


(b) Proliferation inhibition (%)

Compound 1 (μM) across; GDC-1971 (μM) down

| GDC-1971 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | -4.0 | 11.0 | 21.6 | 38.9 | 54.2 | 66.0 | 73.5 | 77.5 | 86.7 |
| 0.0080 | 13.6 | 25.9 | 28.3 | 30.6 | 40.8 | 60.6 | 72.6 | 76.9 | 82.2 | 90.2 |
| 0.0400 | 21.9 | 24.0 | 29.2 | 39.6 | 53.2 | 67.2 | 77.9 | 77.9 | 88.6 | 92.5 |
| 0.2000 | 29.0 | 33.1 | 34.9 | 48.8 | 59.9 | 70.9 | 77.6 | 88.9 | 92.4 | 94.5 |
| 1.0000 | 35.0 | 30.5 | 41.3 | 52.4 | 62.1 | 77.1 | 85.4 | 91.4 | 95.1 | 97.0 |
| 5.0000 | 55.3 | 48.2 | 50.2 | 60.7 | 63.8 | 78.2 | 82.6 | 89.8 | 92.1 | 92.2 |

(c) Excess over Bliss score

Compound 1 (μM) across; GDC-1971 (μM) down

| GDC-1971 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0080 | 0 | 16 | 5 | -2 | -6 | 0 | 2 | 0 | 2 | 2 |
| 0.0400 | 0 | 5 | -1 | 1 | 1 | 3 | 5 | -1 | 6 | 3 |
| 0.2000 | 0 | 7 | -2 | 4 | 3 | 3 | 2 | 8 | 8 | 4 |
| 1.0000 | 0 | -2 | -1 | 3 | 2 | 7 | 8 | 9 | 10 | 6 |
| 5.0000 | 0 | -5 | -10 | -4 | -9 | -1 | -2 | 2 | 2 | -2 |

# Fig.15

(a) Isobologram

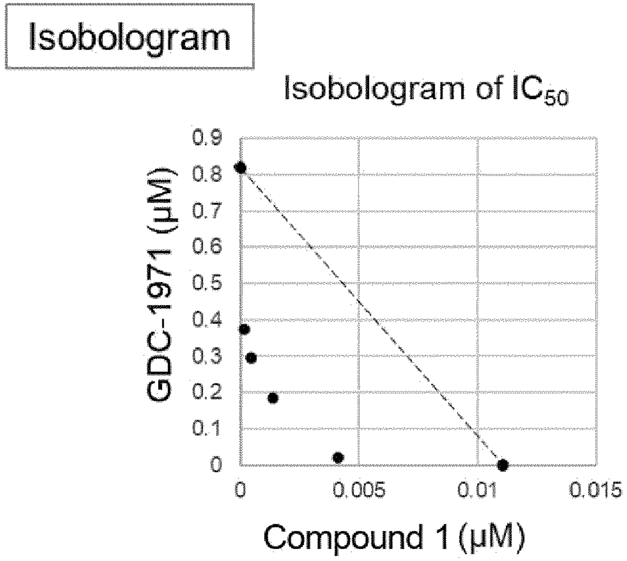


(b) Proliferation inhibition (%)

| GDC-1971 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0.0 | 5.3 | 10.5 | 11.7 | 31.7 | 53.9 | 58.5 | 63.4 | 63.9 | 72.5 |
| 0.0080 | 17.2 | 25.9 | 24.7 | 32.5 | 46.2 | 58.8 | 64.6 | 72.0 | 68.7 | 68.9 |
| 0.0400 | 31.1 | 38.1 | 37.3 | 43.4 | 52.4 | 63.4 | 73.0 | 73.8 | 73.0 | 71.0 |
| 0.2000 | 40.6 | 47.3 | 48.8 | 50.3 | 62.0 | 70.6 | 73.5 | 73.8 | 76.3 | 75.9 |
| 1.0000 | 46.8 | 53.0 | 55.3 | 59.6 | 67.6 | 72.8 | 79.1 | 75.3 | 78.5 | 74.1 |
| 5.0000 | 69.9 | 70.7 | 71.4 | 74.6 | 76.3 | 80.2 | 78.8 | 79.1 | 79.6 | 79.9 |

(c) Excess over Bliss score

| GDC-1971 (μM) \ Compound 1 (μM) | 0.0000 | 0.0002 | 0.0005 | 0.0014 | 0.0041 | 0.0123 | 0.0370 | 0.1111 | 0.3333 | 1.0000 |
|---|---|---|---|---|---|---|---|---|---|---|
| 0.0000 | 0 | 0 | 0 | 0 | 0 | 0 | 0 | 0 | 0 | 0 |
| 0.0080 | 0 | 4 | -1 | 6 | 3 | -3 | -1 | 2 | -1 | -8 |
| 0.0400 | 0 | 3 | -1 | 4 | -1 | -5 | 2 | -1 | -2 | -10 |
| 0.2000 | 0 | 4 | 2 | 3 | 3 | -2 | -2 | -4 | -2 | -8 |
| 1.0000 | 0 | 3 | 3 | 7 | 4 | -3 | 1 | -5 | -2 | -11 |
| 5.0000 | 0 | -1 | -2 | 1 | -3 | -6 | -9 | -10 | -10 | -12 |

# Fig.16

(a) Isobologram

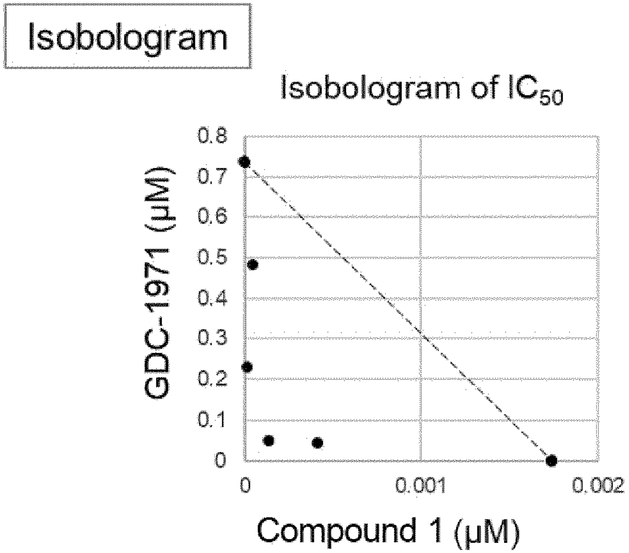


(b) Proliferation inhibition (%)

Compound 1 (μM)

| GDC-1971 (μM) | 0.0000 | 0.00002 | 0.00005 | 0.0001 | 0.0004 | 0.0012 | 0.0037 | 0.0111 | 0.0333 | 0.1000 |
|---|---|---|---|---|---|---|---|---|---|---|
| 0.0000 | 0.0 | -9.8 | -1.4 | 1.6 | 7.6 | 39.7 | 70.0 | 84.8 | 89.8 | 87.2 |
| 0.0080 | -3.1 | 0.4 | -0.5 | 11.6 | 32.6 | 58.3 | 77.3 | 85.7 | 89.2 | 90.4 |
| 0.0400 | 7.4 | 12.3 | 10.4 | 38.5 | 45.1 | 68.7 | 83.7 | 87.4 | 89.3 | 89.9 |
| 0.2000 | 16.7 | 28.2 | 31.5 | 58.1 | 74.2 | 78.3 | 85.8 | 89.6 | 91.3 | 91.9 |
| 1.0000 | 52.4 | 59.4 | 62.0 | 61.0 | 75.3 | 82.3 | 88.2 | 88.0 | 92.1 | 92.7 |
| 5.0000 | 51.7 | 46.5 | 50.7 | 54.1 | 70.0 | 78.3 | 84.4 | 87.9 | 89.6 | 90.0 |

(c) Excess over Bliss score

Compound 1 (μM)

| GDC-1971 (μM) | 0.0000 | 0.00002 | 0.00005 | 0.0001 | 0.0004 | 0.0012 | 0.0037 | 0.0111 | 0.0333 | 0.1000 |
|---|---|---|---|---|---|---|---|---|---|---|
| 0.0000 | 0 | 0 | 0 | 0 | 0 | 0 | 0 | 0 | 0 | 0 |
| 0.0080 | 0 | 14 | 4 | 13 | 28 | 20 | 8 | 1 | 0 | 4 |
| 0.0400 | 0 | 14 | 4 | 30 | 31 | 25 | 11 | 2 | -1 | 2 |
| 0.2000 | 0 | 20 | 16 | 40 | 51 | 29 | 11 | 2 | 0 | 3 |
| 1.0000 | 0 | 12 | 10 | 8 | 19 | 11 | 2 | -5 | -3 | -1 |
| 5.0000 | 0 | 0 | 0 | 2 | 15 | 7 | -1 | -5 | -5 | -4 |

Fig.17

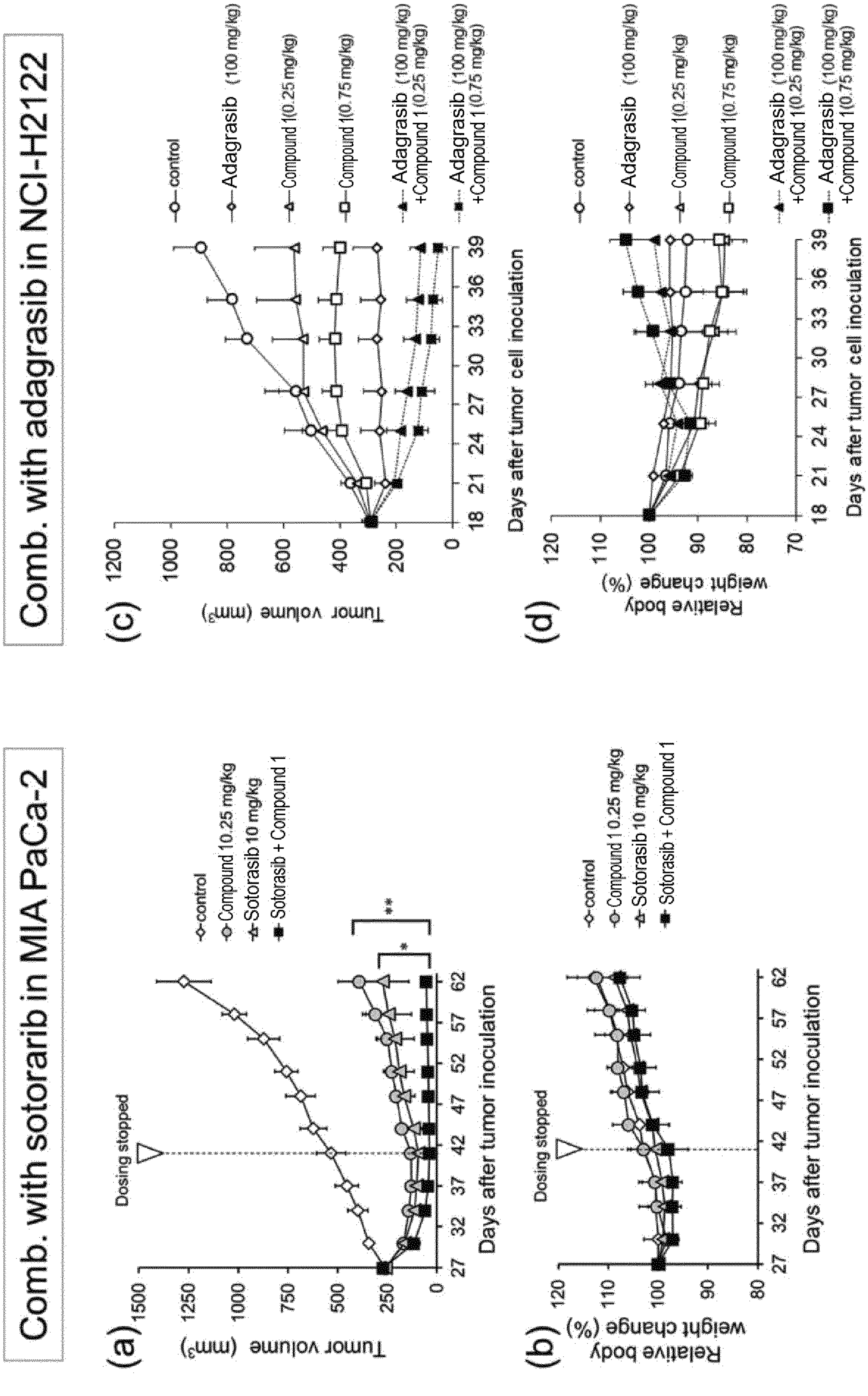
Comb. with sotorarib in MIA PaCa-2
(a)
Tumor volume (mm3)
Dosing stopped
Days after tumor inoculation
control
Compound 1 0.25 mg/kg
Sotorasib 10 mg/kg
Sotorasib + Compound 1
(b)
Relative body weight change (%)
Dosing stopped
Days after tumor inoculation
control
Compound 1 0.25 mg/kg
Sotorasib 10 mg/kg
Sotorasib + Compound 1
Comb. with adagrasib in NCI-H2122
(c)
Tumor volume (mm3)
Days after tumor cell inoculation
control
Adagrasib (100 mg/kg)
Compound 1(0.25 mg/kg)
Compound 1(0.75 mg/kg)
Adagrasib (100 mg/kg) +Compound 1 (0.25 mg/kg)
Adagrasib (100 mg/kg) +Compound 1 (0.75 mg/kg)
(d)
Relative body weight change (%)
Days after tumor cell inoculation
control
Adagrasib (100 mg/kg)
Compound 1(0.25 mg/kg)
Compound 1(0.75 mg/kg)
Adagrasib (100 mg/kg) +Compound 1 (0.25 mg/kg)
Adagrasib (100 mg/kg) +Compound 1 (0.75 mg/kg)

# Fig.18

(a)

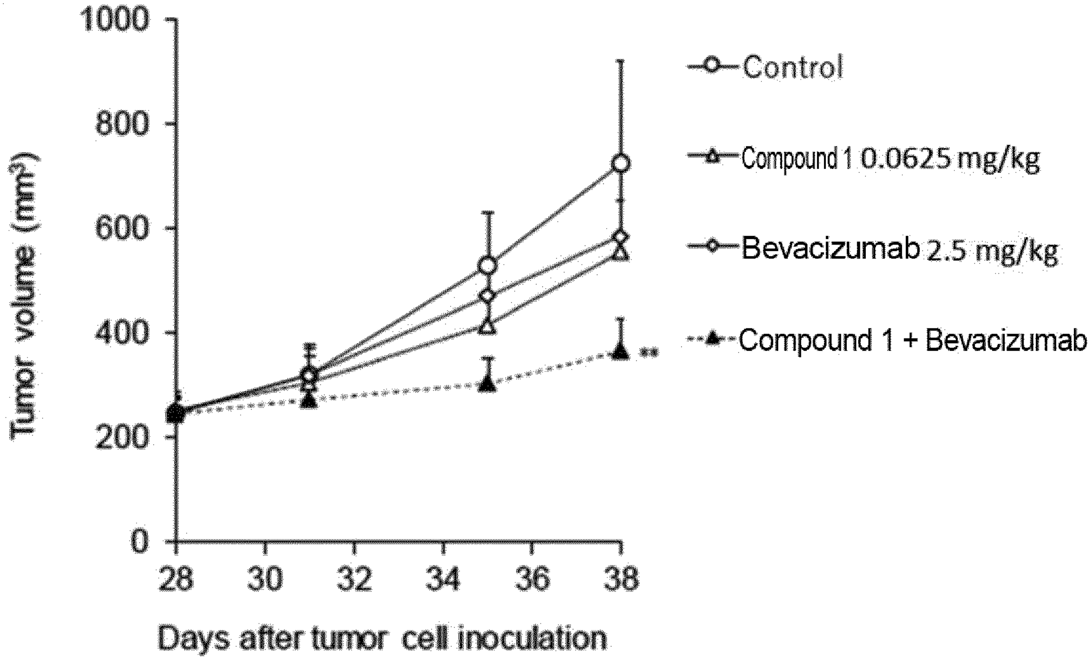


Points, mean for 5 animals; vertical bars, S.D.
** $p<0.005$ vs the Bevacizumab group (Student's t-test).

(b)

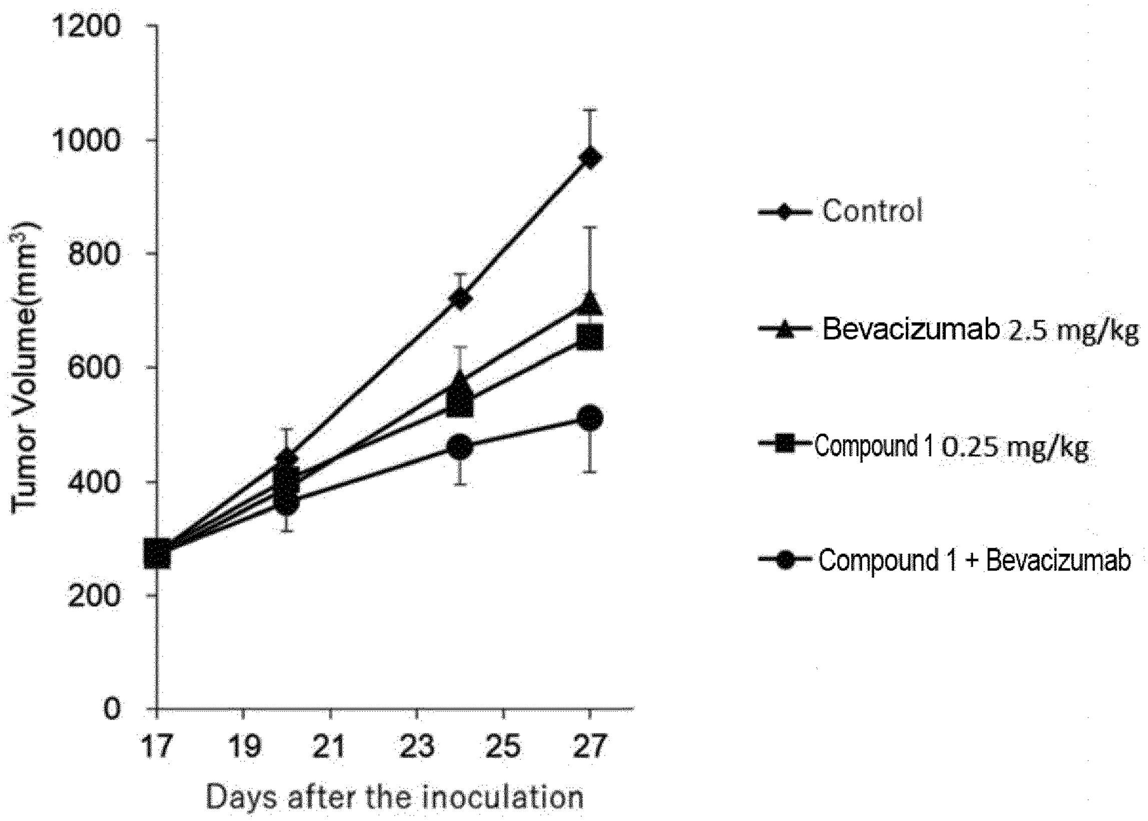

# Fig.19A

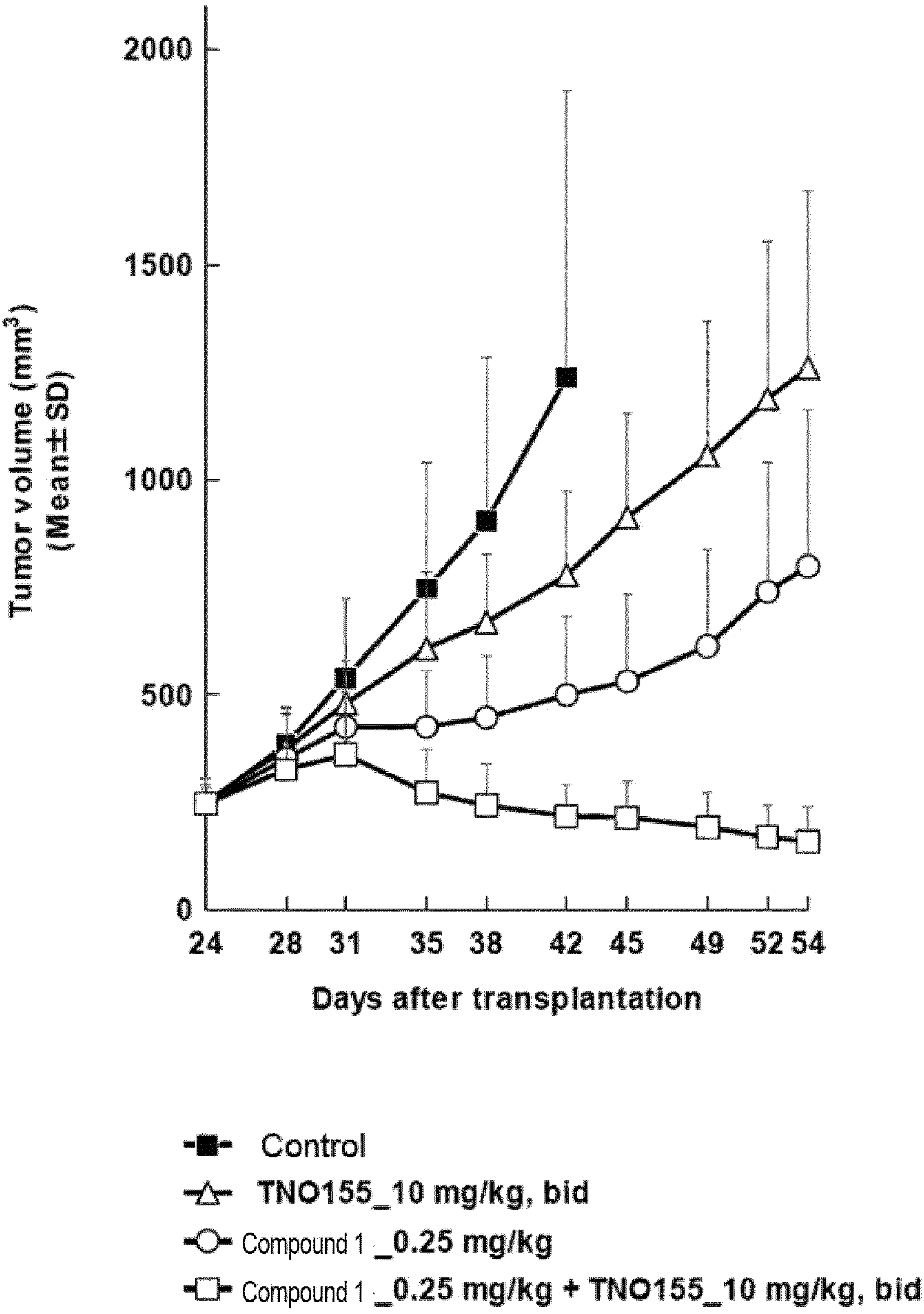
2000
1500
1000
500
0
Tumor volume (mm3)
(Mean±SD)
24
28
31
35
38
42
45
49
52
54
Days after transplantation
Control
TNO155_10 mg/kg, bid
Compound 1 _0.25 mg/kg
Compound 1 _0.25 mg/kg + TNO155_10 mg/kg, bid

*Fig.19B*

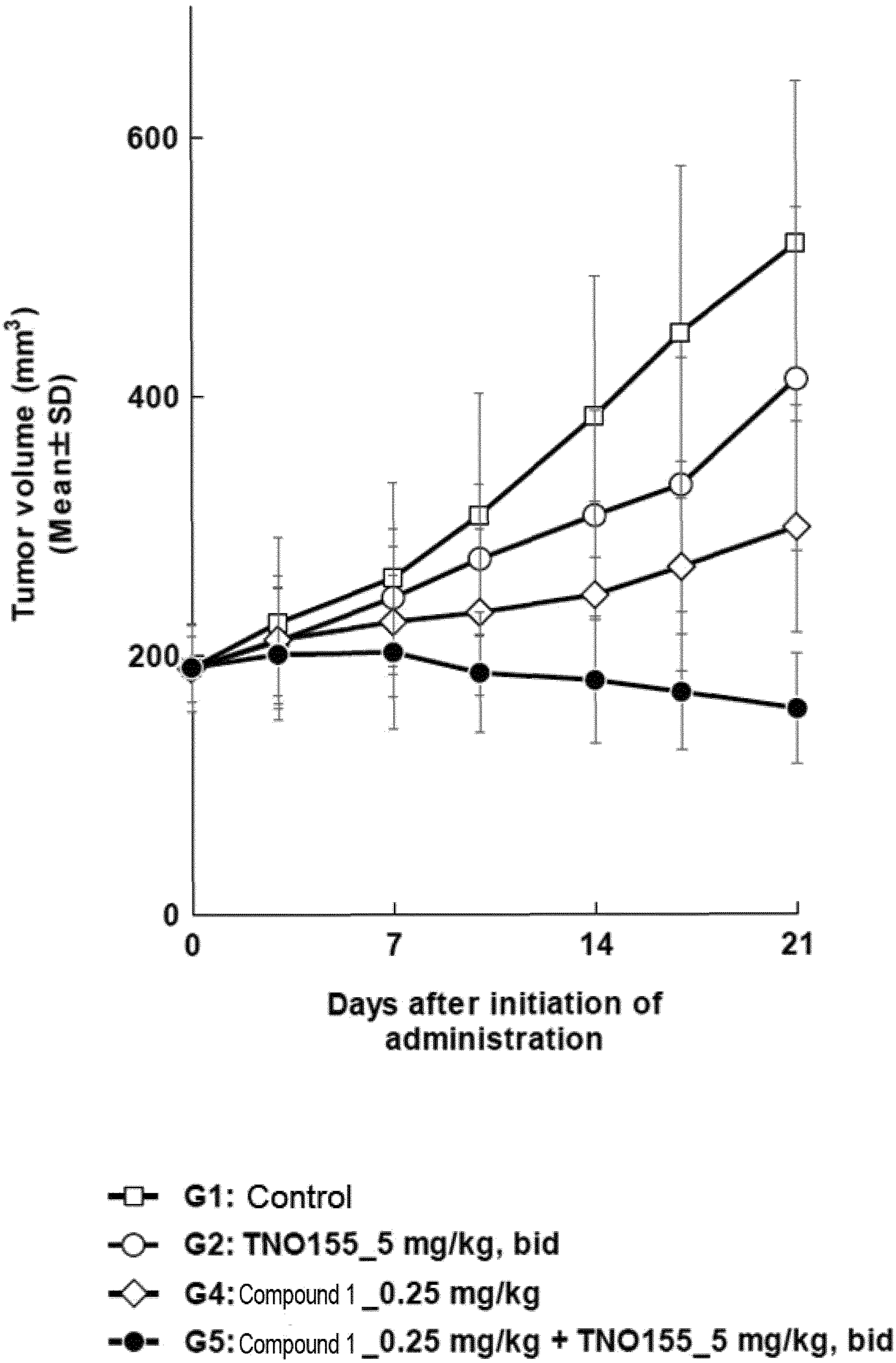
600
400
200
0
Tumor volume (mm3)
(Mean±SD)
0
7
14
21
Days after initiation of administration
G1: Control
G2: TNO155_5 mg/kg, bid
G4: Compound 1 _0.25 mg/kg
G5: Compound 1 _0.25 mg/kg + TNO155_5 mg/kg, bid

Fig.19C

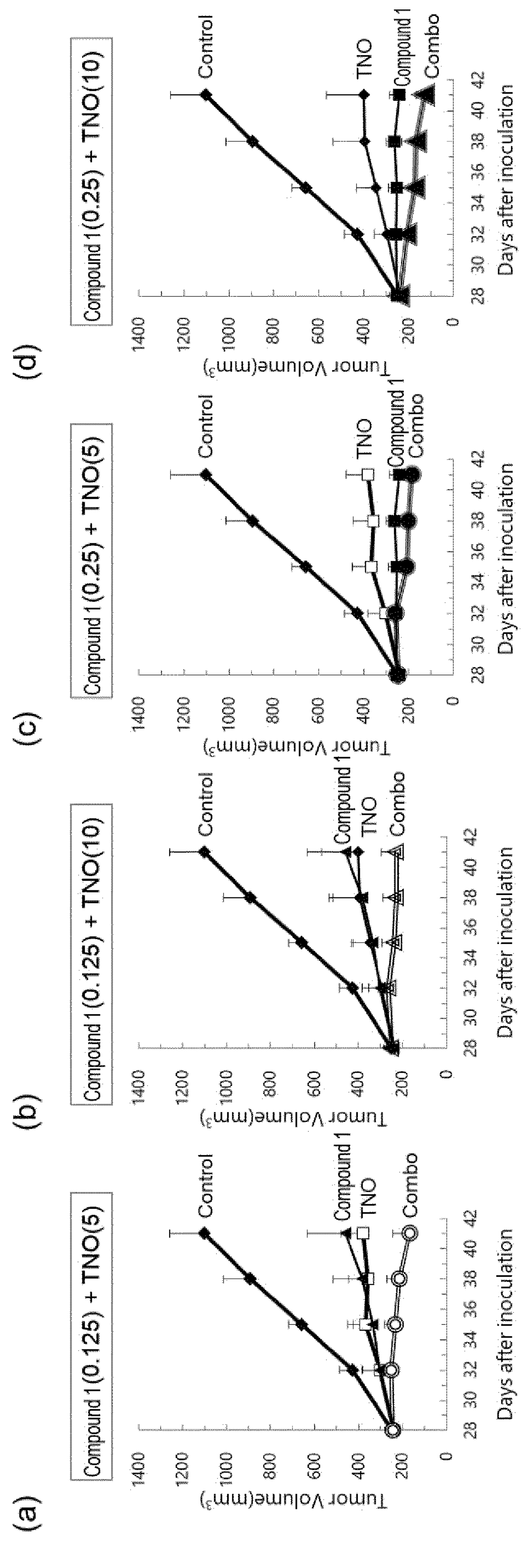
(a)
Compound 1(0.125) + TNO(5)
(b)
Compound 1(0.125) + TNO(10)
(c)
Compound 1(0.25) + TNO(5)
(d)
Compound 1(0.25) + TNO(10)
Tumor Volume(mm³)
Days after inoculation
1400
1200
1000
800
600
400
200
0
28 30 32 34 36 38 40 42
Control
Compound 1
TNO
Combo

Fig.20

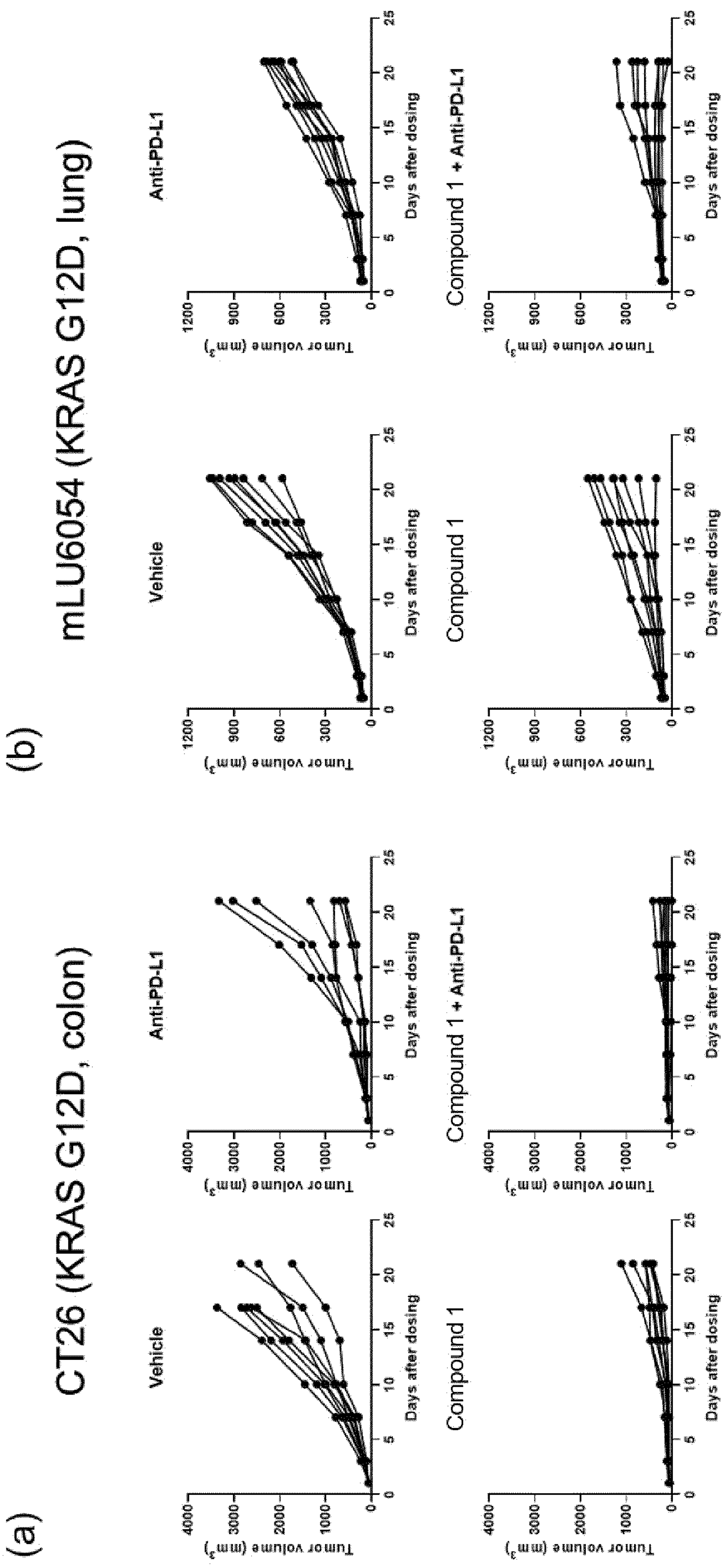
(a)
CT26 (KRAS G12D, colon)
Vehicle
Anti-PD-L1
Compound 1
Compound 1 + Anti-PD-L1
Tumor volume (mm³)
Days after dosing
(b)
mLU6054 (KRAS G12D, lung)
Vehicle
Anti-PD-L1
Compound 1
Compound 1 + Anti-PD-L1
Tumor volume (mm³)
Days after dosing

**INTERNATIONAL SEARCH REPORT**

International application No. **PCT/JP2023/015683**

**A. CLASSIFICATION OF SUBJECT MATTER**

***A61K 31/44***(2006.01)i; ***A61K 31/519***(2006.01)i; ***A61K 45/00***(2006.01)i; ***A61P 35/00***(2006.01)i; ***A61P 35/02***(2006.01)i; ***A61P 43/00***(2006.01)i
FI: A61K31/44; A61P35/00; A61P43/00 121; A61K45/00; A61P43/00 111; A61P35/02; A61K31/519

According to International Patent Classification (IPC) or to both national classification and IPC

**B. FIELDS SEARCHED**

Minimum documentation searched (classification system followed by classification symbols)

A61K31/44; A61K31/519; A61K45/00; A61P35/00; A61P35/02; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2022/019329 A1 (CHUGAI PHARMACEUTICAL CO., LTD.) 27 January 2022 (2022-01-27)<br>paragraph [0142] | 1-18 |
| A | WO 2022/018875 A1 (CHUGAI PHARMACEUTICAL CO., LTD.) 27 January 2022 (2022-01-27)<br>paragraphs [0228], [0229], [0462]-[0464] | 1-18 |
| A | WO 2021/149776 A1 (CHUGAI PHARMACEUTICAL CO., LTD.) 29 July 2021 (2021-07-29)<br>paragraph [0142] | 1-18 |
| A | SHINDE, R., et al., Abstract CT143: Phase I study of the combination of a RAF-MEK inhibitor CH5126766 and FAK inhibitor defactinib in an intermittent dosing schedule with expansions in KRAS mutant cancers, Cancer Res, 2020, 80 (16_Supplement)<br>CT143 | 1-18 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2023** | **18 July 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/015683**

**C. DOCUMENTS CONSIDERED TO BE RELEVANT**

| Category* | Citation of document, with indication, where appropriate, of the relevant passages | Relevant to claim No. |
|---|---|---|
| A | ONO, H., et al., Novel RAF/MEK inhibitor CH5126766/VS-6766 has efficacy in combination with eribulin for the treatment of triple-negative breast cancer, Cancer Sci., 2021, 12(10), 4166-4175<br>fig. 3-6 | 1-18 |

Form PCT/ISA/210 (second sheet) (January 2015)

**INTERNATIONAL SEARCH REPORT**
**Information on patent family members**

International application No.
**PCT/JP2023/015683**

| Patent document cited in search report | Publication date (day/month/year) | Patent family member(s) | Publication date (day/month/year) |
|---|---|---|---|
| WO 2022/019329 A1 | 27 January 2022 | KR 10-2023-0003296 A<br>paragraph [0935] | |
| WO 2022/018875 A1 | 27 January 2022 | KR 10-2022-0012195 A<br>paragraphs [0753], [2887]-[2890] | |
| WO 2021/149776 A1 | 29 July 2021 | US 2023/0002333 A1<br>paragraph [0961]<br>EP 4094804 A1<br>paragraph [0163] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018076369 A
- JP 2021063014 A
- WO 2021149776 A


### Non-patent literature cited in the description


- **S.I. OU et al.** A12 The SHP2 Inhibitor RMC-4630 in Patients with KRAS-Mutant Non-Small Cell Lung Cancer: Preliminary Evaluation of a First-in-Man Phase 1 Clinical Trial. *Journal of Thoracic Oncology*, February 2020, vol. 15 (2), S15-S16
- *CHEMICAL ABSTRACTS*, 2677856-11-8
- *CHEMICAL ABSTRACTS*, 2296729-00-3
- *CHEMICAL ABSTRACTS*, 2326521-71-3
- *CHEMICAL ABSTRACTS*, 2172651-73-7
- *CHEMICAL ABSTRACTS*, 1801765-04-7
- *CHEMICAL ABSTRACTS*, 1801747-42-1
- *CHEMICAL ABSTRACTS*, 56990-57-9
- *CHEMICAL ABSTRACTS*, 284461-73-0
- *CHEMICAL ABSTRACTS*, 444731-52-6
- *CHEMICAL ABSTRACTS*, 557795-19-4
- *CHEMICAL ABSTRACTS*, 319460-85-0
- *CHEMICAL ABSTRACTS*, 755037-03-7
- *CHEMICAL ABSTRACTS*, 216974-75-3
- *CHEMICAL ABSTRACTS*, 2377352-49-1
- *Cancer Metastasis Rev*, 2013, vol. 32, 147-162
- *Cancer Discov.*, 2016, vol. 6, 316-329
- *Nat. Rev. Drug Discov.*, 2020, vol. 19, 533-552
- *Molecular Cytogenetics*, 2015, vol. 8, 103